# EUROPEAN PATENT APPLICATION

(11) **EP 1 992 694 A1**
(43) Date of publication of application: **19.11.2008**
(21) Application number: 07108349.7
(22) Date of filing: 16.05.2007
(51) Int. Cl.: C12N 15/11, C12N 15/12, C12N 5/10, C07K 14/47, C12Q 1/68, A61K 38/17

(54) **Anti-tumor drug, medicament, composition, and use thereof**

(71) Applicant: GENE SIGNAL INTERNATIONAL SA, Epalinges, VD (CH)
(72) Inventor: Al Mahmood, Salman, 75014, PARIS (FR); Colin, Sylvie, 75014, PARIS (FR)
(74) Representative: Cabinet Plasseraud

(57) **Abstract**

The present invention relates to an active polypeptide comprising the amino acid sequence of SEQ ID NO:3, or having at least 50%, preferably 70%, more preferably 90% identity with the amino acid sequence of SEQ ID NO:3, or fragments thereof having at least 20 contiguous amino acids, or peptides having at least 50%, preferably 70%, more preferably 90% identity with the amino acid sequence of said fragments, provided that said polypeptide is not SEQ ID NO:2.

## Description

The present invention relates to the field of treatments for cancers. More specifically, the present invention relates to the treatment of cancers by small polypeptides.

Cancer is a class of diseases or disorders characterized by uncontrolled division of cells and the ability of these cells to spread, either by direct growth into adjacent tissue through invasion, or by implantation into distant sites by metastasis (where cancer cells are transported through the bloodstream or lymphatic system). Cancer may affect people at all ages, but risk tends to increase with age. It is one of the principal causes of death in developed countries.

There are many types of cancer. Severity of symptoms depends on the site and character of the malignancy and whether there is metastasis. Once diagnosed, cancer is usually treated with a combination of surgery, chemotherapy and radiotherapy. As research develops, treatments are becoming more specific for the type of cancer pathology. Drugs that target specific cancers already exist for several cancers. If untreated, cancers may eventually cause illness and death, though this is not always the case.

Current treatments target distinct properties of malignant cells, such as for example evading apoptosis, unlimited growth potential (immortalization) due to overabundance of telomerase, self-sufficiency of growth factors, insensitivity to anti-growth factors, increased cell division rate, altered ability to differentiate, no ability for contact inhibition, ability to invade neighbouring tissues, ability to build metastases at distant sites, ability to promote blood vessel growth (angiogenesis).

Tumor angiogenesis is the proliferation of a network of blood vessels that penetrates into the tumor, supplying nutrients and oxygen and removing waste products. Tumor angiogenesis actually starts with cancerous tumor cells releasing molecules that send signals to surrounding normal host tissue. This signalling activates certain genes in the host tissue that, in turn, make proteins to encourage growth of new blood vessels. Solid tumors must stimulate the formation of new blood vessels in order to obtain the nutrients and oxygen necessary for their growth, thus providing a route by which the tumors can metastasize to distant sites.

Experimental evidence has suggested that malignant tumors can induce angiogenesis through the elaboration of a variety of factors, such as acidic fibroblast growth factor (aFGF), basic fibroblast growth factor (bFGF), vascular endothelial growth factor (VEGF), platelet derived growth factor (PDGF), transforming growth factor alpha (TGF-alpha), tumor necrosis growth factor alpha (TNF-alpha), and many others *(*Liotta et al., 1991, Cell 64: 327-336*;* Hanahan et al., Cell 86: 353-364*).*

Nowadays, plenty of chemotherapeutic molecules targeting angiogenesis are available on the market. Well known naturally occurring angiogenesis inhibitors are angiostatin, endostatin, interferons, platelet factor 4, prolactin 16Kd fragment, thrombospondin, TIMP-1 (tissue inhibitor of metalloprotease -1), TIMP-2 and TIMP-3. These molecules can be used as chemotherapeutic treatments, as well as other drugs such as for example combrestatin A4, EMD 121974, TNP-470, squalamine, thalidomide, interferon-alpha, anti-VEGF, antibodies... However, their efficiency is never sufficient and alternative treatments are desirable.

There is therefore a need of alternative chemotherapeutic agents for the treatment of tumors, having increased efficiency, being less invasive or toxic, and resulting in an increased rate of recovery.

WO 03/080105, in the name of the Applicant, describes five genes involved in the regulation of angiogenesis. Amongst these genes, "gene 168" (SEQ ID N°1 in this specification), which encodes "protein 168A" (SEQ ID N°2 in this specification), has been described as implied in the activation of angiogenesis. In particular, WO 03/080105 discloses that protein 168A is expressed in endothelial cells stimulated with pro-angiogenic factors, such as for example TNF-α. WO 03/080105 also describes that the expression, in human endothelial cells, of an antisens sequence of gene 168, i.e. the inhibition of the expression of gene 168, inhibits the formation of capillary tubes.

*In silico* experiments further revealed that protein 168A, which is constituted of 924 aminoacids, may have a single transmembrane domain, and five Immunoglobulin-like domains.

Going deeper in their researches, the inventors produced truncated forms of protein 168A, corresponding to various fragments of protein 168A. Amongst these fragments, 168A-T2 corresponds to a fragment of the extracellular domain of protein 168A, and is identified by SEQ ID NO :4 in this specification (108 amino acids).

In a first experiment, the inventors found that protein 168A-T2 may inhibit human endothelial cell proliferation *in vitro* in a dose dependent manner.

Then, in a second experiment, the inventors surprisingly found that 168A-T2 may have a strong activity to inhibit capillary tube formation *in vitro,* in a dose dependent manner.

Other experiments conducted by the inventors suggested that 168A-T2 may induce the inhibition of the migration of endothelial cells *in vitro,* in a dose dependent manner.

The results of the dose-response study further revealed that the protein 168A-T2 may inhibit in a dose-dependent manner the proliferation of human endothelial cells, and this inhibition could reach more than 80% with a concentration of 3.1 µM. This tends to demonstrate that the recombinant protein may be a potent anti-angiogenic compound, at least 600-fold more potent than the anti-VEGF mAb and/or VEGF receptor (KDR)-based identified peptides (Binetruy-Tournaire R et al., Identification of a peptide blocking vascular endothelial growth factor (VEGF)-mediated angiogenesis, EMBO J. 2000; 19: 1525-1533*).*

Capillary tube formation, human endothelial cell proliferation and human endothelial cell migration are three essential steps of angiogenesis. Consequently, the fact that 168A-T2 may inhibit *in vitro* capillary tube formation, human endothelial cell proliferation and/or migration in a dose-dependant manner, thus constituted a strong evidence of the potent anti-angiogenic activity of the truncated forms of protein 168A.

All these results were absolutely unexpected since native protein 168A is preferably expressed in pro-angiogenic conditions, i.e. in the presence of TNFα, and that the expression of an antisens of the gene 168, i.e. the inhibition of gene 168, in human endothelial cells inhibits the formation of capillary tubes. It was therefore really surprising that a truncated form of protein 168A, may have anti-angiogenic activity.

Still surprisingly, the inventors found that protein 168A-T2 had a strong anti-tumor activity *in vivo,* and a strong synergistic activity in combination with other chemotherapeutic agent such as for example cisplatin.

Inventors found that the test substance 168A-T2 was not toxic in Nude mice bearing tumors at different tested doses. Moreover, 168A-T2 exhibited a strong statistically significant anti-tumoral activity against human tumors as early as two days after the beginning of the treatment. This anti-tumoral activity was persistent during the treatment period. This anti-tumoral effect of 168A-T2 represented a realistic therapeutic approach as a monotherapy. Its efficacy was also strongly potentiated when combined with the cytotoxic anticancer drug CDDP (Cisplatin), which suppressed tumor growth. Cisplatin alone, on the other hand, did not eradicate tumor growth.

The data strongly suggested that 168A-T2 may be of use either as a primary anti-tumoral agent or as an add-on synergic therapy to primary cytotoxic agents for the treatment of cancers.

As mentioned above, it is now established that protein 168A is expressed in endothelial cells, and that its expression is enhanced when angiogenesis is stimulated by pro-angiogenic factors such as TNFα, as described in WO03/080105. Protein 168A is a transmembrane protein, and might be implied in the transduction of a pro-angiogenic signal. Without wanting to be bound with a theory, Applicants suggest that the truncated forms of 168A may play their role through a "soluble receptor mechanism": truncated forms of 168A may remain soluble on the surface of the cell and may be recognized by the ligand of the native 168A protein. As a result, there may be a competition in the recognition of the ligand between the soluble forms of 168A (the fragments of the invention) and the native transmembrane protein, and consequently a decrease, in a dose dependent manner, of the transduction of the pro-angiogenic signal, therefore resulting in the inhibition of angiogenesis and then in the decrease of tumour volume.

The invention thus relates, in a first aspect, to a nucleic acid having the nucleic acid sequence of SEQ ID NO:3, or having at least 50%, preferably 70%, more preferably 90% identity with the nucleic acid sequence of SEQ ID NO:3, or fragments thereof having at least 60 contiguous nucleotides, or nucleic acid sequences having at least 50%, preferably 70%, more preferably 90% identity with the nucleic acid sequence of said fragments, provided that said nucleic acid is not SEQ ID NO:1, said nucleic acid coding for a polypeptide or peptide having anti-tumour activity.

As used above, "fragments" means truncated sequences of SEQ ID NO:3, having at least 60 contiguous nucleotides, and coding for an active peptide or polypeptide. In a particular embodiment, the fragments have the nucleic acid sequence of SEQ ID NO:13, SEQ ID:14, SEQ ID NO:15, SEQ ID NO:16, SEQ ID NO:17, or SEQ ID NO:18.

In another aspect, the invention relates to an expression vector comprising at least one nucleic acid sequence as defined above.

As used herein, "expression vector" means any plasmid or nucleic acid construct, which is used to introduce and express a specific nucleic acid sequence into a target cell.

The invention further relates, in a third aspect, to an active polypeptide comprising the amino acid sequence of SEQ ID NO:4, or having at least 50%, preferably 70%, more preferably 90% identity with the amino acid sequence of SEQ ID NO:4, or fragments thereof having at least 20 contiguous amino acids, or peptides having at least 50%, preferably 70%, more preferably 90% identity with the amino acid sequence of said fragments, provided that said polypeptide is not SEQ ID NO:2.

As used herein, "peptide" means short molecules formed from the linking, in a defined order, of less than 100 amino acids.

As used herein, "polypeptide" means molecules formed from the linking, in a defined order, of at least 100 amino acids.

As used herein, "active polypeptide" means polypeptides which have a biological activity. In the present invention the polypeptides have an anti angiogenic and anti tumour activity.

According to the invention, the polypeptide as described above has an anti-tumour activity.

According to the invention, the fragments of the polypeptide as described above correspond to truncated forms of the protein identified by SEQ ID NO:4, and having an anti-tumor activity. Said fragments preferably have an amino acid sequence of at least 20 contiguous amino acids of SEQ ID NO:4. In a particular embodiment, the fragments have an amino acid sequence of at least 37 contiguous amino acids. In another particular embodiment, said fragments have the amino acid sequence of SEQ ID N:7 (90 amino acids), SEQ ID N:8 (77 amino acids), SEQ ID N:9 (66 amino acids), SEQ ID N:10 (51 amino acids), SEQ ID N:11 (37 amino acids) or SEQ ID N:12 (20 amino acids). Fragments also include peptides having at least 50%, preferably 70%, more preferably 90% identity with the amino acid sequence of said fragments, and having an anti-tumor activity.

In a particular embodiment, the active polypeptides according to the invention are produced by the expression vector as defined above.

In a fourth aspect, the present invention relates to a medicament comprising at least one nucleic acid sequence, vector, or polypeptide as described above.

In a fifth aspect, the invention relates to a pharmaceutical composition comprising at least one nucleic acid sequence, vector, or polypeptide as described above, and one or more pharmaceutically acceptable excipients.

In a sixth aspect, the invention relates to a pharmaceutical composition comprising at least one nucleic acid sequence, vector, or polypeptide as described above, and one or more pharmaceutically-acceptable excipients, for use in a method of treatment of cancer and/or tumors of the human or animal body.

In a particular embodiment, the pharmaceutical compositions as described above further comprise at least one another active substance selected from anti-angiogenic substances or anti-tumor substances. These substances may be chosen by the man in the art, regarding the effect to be achieved. Preferably, these substances can be selected from cisplatin, carboplatin, etoposide, ifosfamide, mitomycin, vinblastine, vinorelbine, gemcitabine, paclitaxel, docetaxel, and irinotecan, etc...

In a seventh aspect, the invention relates to a pharmaceutical composition comprising effective amounts of
- a polypeptide, a fragment, and/or a peptide as described above, and
- a platinum complex selected from the group consisting of cisplatin and carboplatin.

Applicants surprisingly found that the combination of a polypeptide according to the invention with a platinum complex showed synergistic activity.

By "synergistic", it is meant, within the present invention, that the total effect of the combination of active principles is greater than the effect of each active principle taken separately.

The medicament or composition useful in the practice of this invention is administered to the mammal by known conventional routes. The medicament or composition described herein may be administered by the same route, or by different routes. For example, the medicament or composition may be administered to patients orally or parenterally (intravenously, subcutaneously, intramuscularly, intraspinally, intraperitoneally, and the like).

When administered parenterally, the composition is preferably formulated in a unit dosage injectable form (solution, suspension, emulsion) with at least one pharmaceutically acceptable excipient. Such excipients are typically nontoxic and non-therapeutic. Examples of such excipients are water, aqueous vehicles such as saline, Ringer's solution, dextrose solution, and Hank's solution and non-aqueous vehicles such as fixed oils (e.g., corn, cottonseed, peanut and sesame), ethyl oleate, and isopropyl myristate. Sterile saline is a preferred excipient. The excipient may contain minor amounts of additives such as substances that enhance solubility, isotonicity, and chemical stability, e.g., antioxidants, buffers, and preservatives. When administered orally (or rectally) the compounds will usually be formulated into a unit dosage form such as a table, capsule, suppository, or cachet. Such formulations typically include a solid, semi-solid or liquid carrier or diluent. Exemplary diluents and excipients are lactose, dextrose, sucrose, sorbitol, mannitol, starches, gum acacia, calcium phosphate, mineral oil, cocoa butter, oil of theobroma, alginates, tragacanth, gelatin, methylcellulose, polyoxyethylene, sorbitan monolaurate, methyl hydroxybenzoate, propyl hydroxybenzoate, talc and magnesium stearate. In preferred embodiments, the pharmaceutical composition according to the invention is administered intravenously.

According to the invention, the amount of polypeptide present in the medicament or composition is effective to treat susceptible tumors. Preferably, the polypeptide is present in an amount from 0.01 to 90% in weight, preferably from 0.1% to 10% in weight, more preferably from 1% to 5% in weight, in the medicament or in the composition. These amounts are routinely adaptable by the man in the art, who is able to choose the best quantity to administer to a patient to achieve recovery.

In an eight aspect, the invention relates to the use of at least one nucleic acid sequence, vector, or polypeptide as described above, or of the medicament as described above, or of the pharmaceutical composition as described above, for the treatment of cancers and/or tumors.

According to the invention, the tumors to be treated are preferably solid tumors. More preferably, the tumors to be treated are selected from sarcomas, carcinomas, and lymphomas. Examples of such tumors are bladder cancer, melanoma, breast cancer, non-Hodgkin's lymphoma, brain cancer, bone cancer, colon and rectal cancer, liver cancer, pancreatic cancer, endometrial cancer, prostate cancer, kidney cancer, skin cancer (non-melanoma), thyroid cancer, lung cancer (small cell lung cancer and non small cell lung cancer).

In a ninth aspect, the present invention relates to a method of treatment comprising administering to a subject in need of treatment at least one nucleic acid sequence, vector, or polypeptide as described above, or the medicament as described above, or the pharmaceutical composition as described above, in an amount sufficient to inhibit cancer or tumor growth.

As used herein, "subject in need of treatment" means any human or warm blood animal who suffers from cancer or tumour.

In a particular embodiment, the invention relates to the method of treatment as described above further comprising administering at least one other anti-neoplastic or anti-tumor drug.

In these methods, administering comprises topical administration, oral administration, intravenous administration, or intraperitoneal administration.

In an eight aspect, the present invention relates to a method of treatment comprising administering to a subject in need of treatment an effective amount of
- a polypeptide, a fragment, and/or a peptide as described above, and
- a platinum complex selected from the group consisting of cisplatin and carboplatin,
which is sufficient to inhibit cancer or tumor growth.

Applicants surprisingly found that the administration of both a polypeptide according to the invention and a platinum complex showed synergistic effect.

In one embodiment, said polypeptide or fragments thereof and said platinum complex are administered simultaneously.

In another embodiment, said polypeptide or fragments thereof and said platinum complex are administered sequentially. Preferably, said polypeptide or fragments thereof and said platinum complex are administered by separate routes, i.e. orally or parenterally (intravenously, subcutaneously, intramuscularly, intraspinally, intraperitoneally, and the like).

In a particular embodiment, said platinum complex is cisplatin.

In another particular embodiment said platinum complex is carboplatin.

The present invention will now be further described with reference to the following non-limiting

### examples.

Figure 1 is a diagram representing the % inhibition of endothelial cell proliferation *in vitro* with increasing concentrations of protein 168A-T2.

Figures 2a, 2b, 2c and 2d are pictures of *in vitro* angiogenesis of endothelial cells in different conditions:

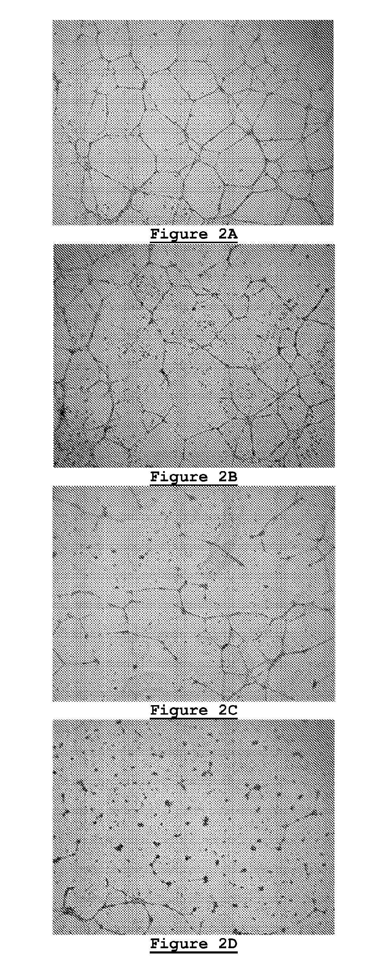

- Figure 2a: Control (Buffer Urea 2M)
- Figure 2b: 168A-T2 3.5µg/mL (0.2µM)
- Figure 2c: 168A-T2 6.9µg/mL (0.4µM)
- Figure 2d: 168A-T2 13.6µg/mL (0.8µM)

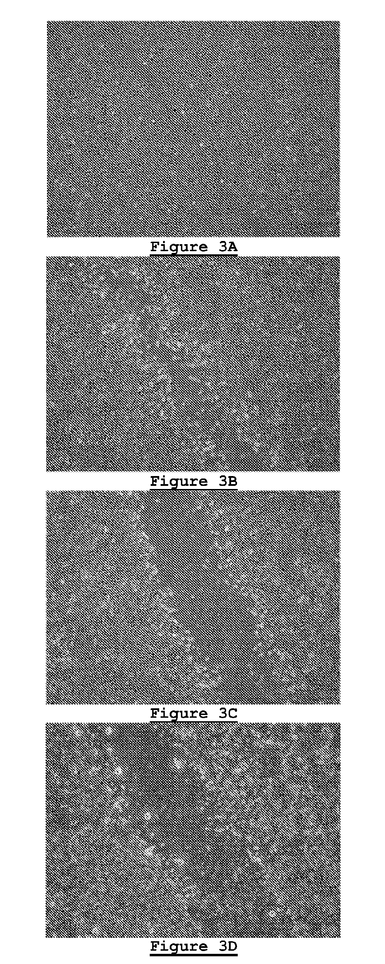

Figures 3a, 3b, 3c and 3d are pictures of wound assay on endothelial cells performed in different conditions:
- Figure 3a: Control (Buffer Urea 2M)
- Figure 3b: 168A-T2 12µg/mL (0.7µM)
- Figure 3c: 168A-T2 17µg/mL (1 µM)
- Figure 3d: 168A-T3 23µg/mL (1.35µM)

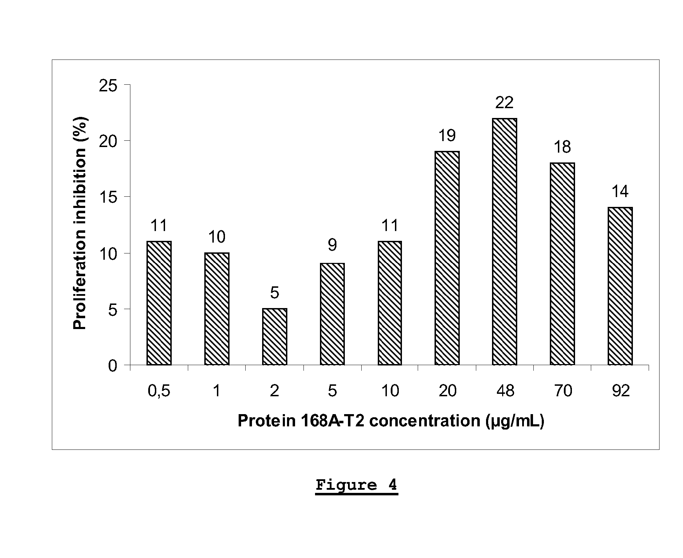

Figure 4 is a diagram representing the % inhibition of kidney tumor cell (Biz) proliferation *in vitro* with increasing concentrations of protein 168A-T2.

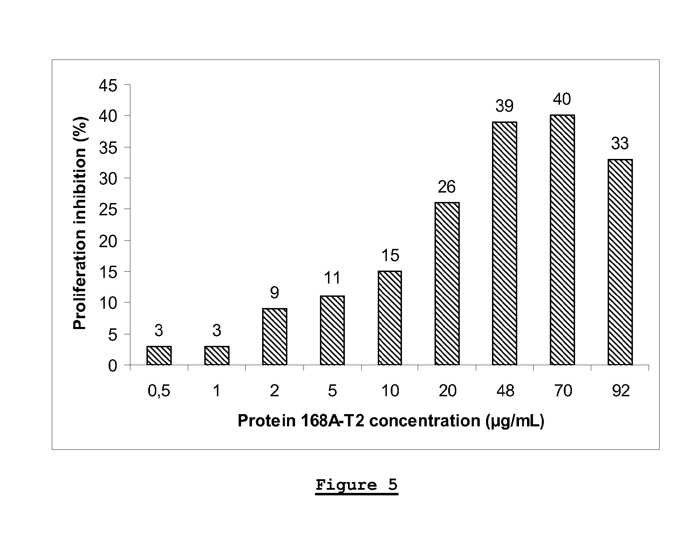

Figure 5 is a diagram representing the % inhibition of lung tumor cell (Calu-6) proliferation *in vitro* with increasing concentrations of protein 168A-T2.

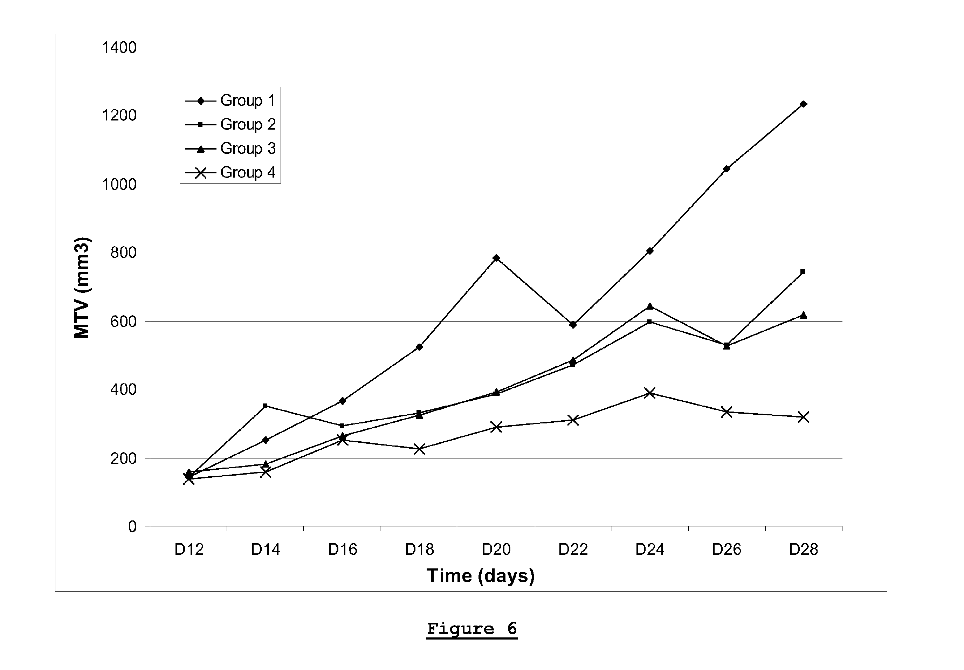

Figure 6 is a graph representing Mean Tumor Volume (mm³) versus time (days) for different groups of mice treated according to example 8.

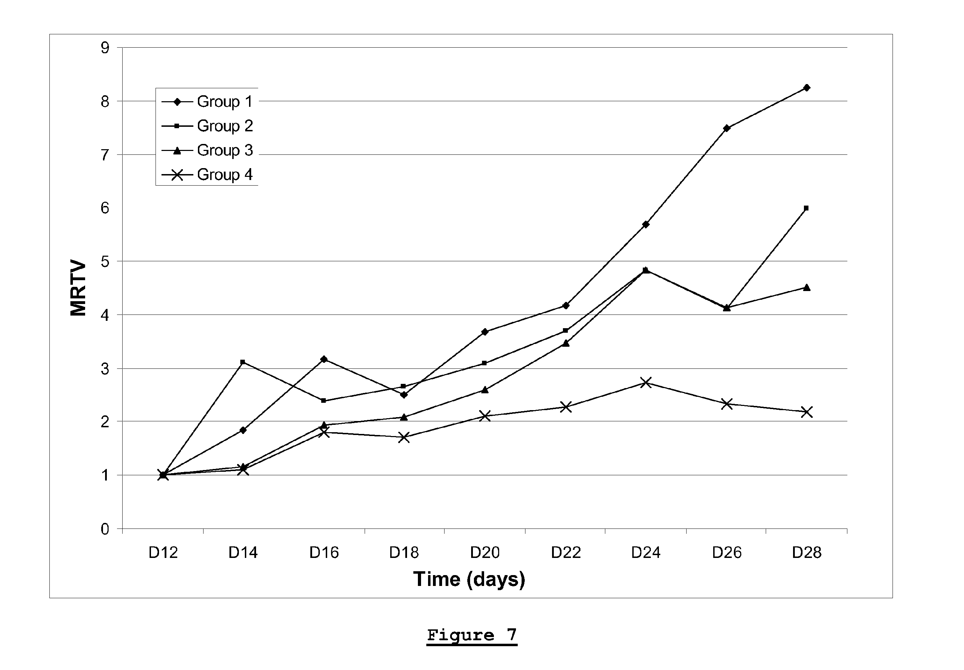

Figure 7 is a graph representing Mean Relative Tumor Volume (without unit) versus time (days) for different groups of mice treated according to example 8.

SEQ ID NO:1 corresponds to the nucleic acid sequence of gene 168.

SEQ ID NO:2 corresponds to the amino acid sequence of protein 168A.

SEQ ID NO:3 corresponds to the nucleic acid sequence of 168A-T2.

SEQ ID NO:4 corresponds to the amino acid sequence of 168A-T2.

SEQ ID NO:5 corresponds to the nucleic acid sequence of 168A-T2 within vector pET30.

SEQ ID NO:6 corresponds to the amino acid sequence of 168A-T2 as produced with the vector pET30.

SEQ ID NO:7 corresponds to the amino acid sequence of a fragment of 168A-T2.

SEQ ID NO:8 corresponds to the amino acid sequence of a fragment of 168A-T2.

SEQ ID NO:9 corresponds to the amino acid sequence of a fragment of 168A-T2.

SEQ ID NO:10 corresponds to the amino acid sequence of a fragment of 168A-T2.

SEQ ID NO:11 corresponds to the amino acid sequence of a fragment of 168A-T2.

SEQ ID NO:12 corresponds to the amino acid sequence of a fragment of 168A-T2.

SEQ ID NO:13 corresponds to the nucleic acid sequence coding for SEQ ID NO:7.

SEQ ID NO:14 corresponds to the nucleic acid sequence coding for SEQ ID NO:8.

SEQ ID NO:15 corresponds to the nucleic acid sequence coding for SEQ ID NO:9.

SEQ ID NO:16 corresponds to the nucleic acid sequence coding for SEQ ID NO:10.

SEQ ID NO:17 corresponds to the nucleic acid sequence coding for SEQ ID NO:11.

SEQ ID NO:18 corresponds to the nucleic acid sequence coding for SEQ ID NO:12.

### EXAMPLE 1: Production of protein 168A-T2

### Synthesis of insert 168A-T2:

First, gene 168A was cloned in pGEM®-T easy vector system (Promega®) according to known procedures (the vector obtained was called "pGEM-T-168A").

Second, the insert T2 (SEQ ID NO:3), coding for the plasma membrane adjacent part of the extra-cellular domain of the protein 168A, was amplified by PCR using the plasmid "pGEM-T-168A" and the two primers CDS5 and CDS4 (table 1).

**Table 1**

| Primer | Sequence |
|---|---|
| 168A-cds-5 | GACGACGACAAGATGGCCTTTGATGTGTCCTGGTTTG |
| 168A-cds-4 | GAGGAGAAGCCCGGTTCAGGGATACTTGAAGGCGTTCAGCACA |

Third, the DNA sequence (SEQ ID NO:3) coding for the protein 168A-T2 was inserted into the vector pET-30 EK/LIC (Novagen®) according to known procedures (pET-30-168A-T2). The nucleic acid sequence coding for 168A-T2 within the pET-30 vector is given in SEQ ID NO:5.

The purified vector was then introduced in *E. coli* BL21(DE3)pLys for protein production. Colonies were controlled for the presence of both the vector end the insert by PCR.

The size of the produced protein 168A-T2 was 18 kD, which corresponded to the expected size, comprising the His-Tag at the N-terminal as confirmed by sequencing. The amino acid sequence of the protein 168A-T2 as produced is given in SEQ ID NO:6.

### Extraction and purification of the protein 168A-T2

As the protein 168A-T2 was produced within the insoluble fraction of the bacteria, it necessitated an extraction in denaturating conditions

Following culture, bacteria were lyzed, centrifuged and the supernatant discarded. The insoluble fraction obtained was treated with Tris-HCl 20 mM, urea 8 M, imidazol 5 mM, NaCl 0.5 M, GSH 5 mM, pH 8.0. After this treatment, the suspension was centrifuged and the supernatant collected, filtered on 0.45µm membranes to discard insoluble materials. The filtered extract was then used to purify the protein 168A-T2 by using a His-Trap column (Amersham®) connected to a HPLC system (Amersham).

The purified protein obtained was diluted in 4 M urea and 0.3 M imidazol. To remove these agents from the preparation, the solution was subjected to dialysis at 4°C.

Following these steps of dialysis, the purified protein was centrifuged at 4,000 x g for 15 min and filtered on 0.45 µm membranes to eliminate possible precipitates. The purified protein preparation was controlled for protein content according to the method described by Bradford in 1976 (Anal. Biochem. 72:248-54) and by SDS-PAGE. The gels were analyzed using the Gene Genius software to quantify the purity by image analysis.

To increase purity of the protein 168A-T2, we performed a second purification step by using ion exchange liquid chromatography. The HisTrap purified preparation was diluted 3 times with the buffer Tris-HCl 20 mM, pH 8, 2 M urea (to decrease the concentration of NaCl to 50 mM), and loaded on MonoS column connected to a HPLC system run by Unicorn software (Amersham, GE, Saclay, France). The column was then washed extensively and eluted with a linear gradient of ionic force (0.05 M to 0.5 M NaCl in the Tris-HCl 20 mM buffer, pH 8, 2 M urea). The purified protein preparation was controlled for protein content both by Bradford and by SDS-PAGE.

### EXAMPLE 2: Test of inhibition of endothelial cell proliferation by 168A-T2 in vitro

HUVEC cells were cultured to confluency in complete EGM2-MV medium (Cambrex) at 37°C and in 5% CO₂ humidified atmosphere. Cells were then collected by trypsine-EDTA digestion (Versene, Eurobio). After 5 min, the enzymatic reaction was stopped by adding 3 ml of the culture medium containing 5% FCS. Cells were then centrifuged at 220 g for 10 min at room temperature, washed twice with 5 ml of culture medium, suspended in complete culture medium, counted and adjusted to 50 000 cells/ml. One hundred µL per well were then distributed to a 96-well cell culture grade micro-plate (5 000 cells/well) and incubated with different concentrations of the purified protein 168A-T2 in Tris-HCl 20 mM buffer (pH 8), containing 150mM NaCl and urea 2M; this buffer was used as control.

After 42 hrs at 37°C, cell proliferation was measured using thiazolyl blue tetrazolium bromide (MTT) method. Briefly, MTT (Sigma) was dissolved in PBS at 5 mg/ml, the solution was filtered (0.22 µm) and 10 µl were added to each well of the 96-well micro-plates. After 3 hrs of incubation at 37°C, 5% CO₂ humidified atmosphere, the micro-plates were centrifuged at 220 x g for 10 min, the supernatant was discarded, and the crystals dissolved by the addition of 100 µl of DMSO to each well. The optical density (OD) at 570 nm was then measured using µQuant micro-plate reader (Bio-Tek Instrument gmbh, Colmar, France) coupled to the KC4 (Bio-Tek) software. The OD was corrected by subtracting blank-well OD values (the OD values obtained from wells without cells), and the inhibition of cell proliferation was measured relative to control (OD obtained from wells with untreated HUVEC representing the maximal proliferative response, i.e.100%).

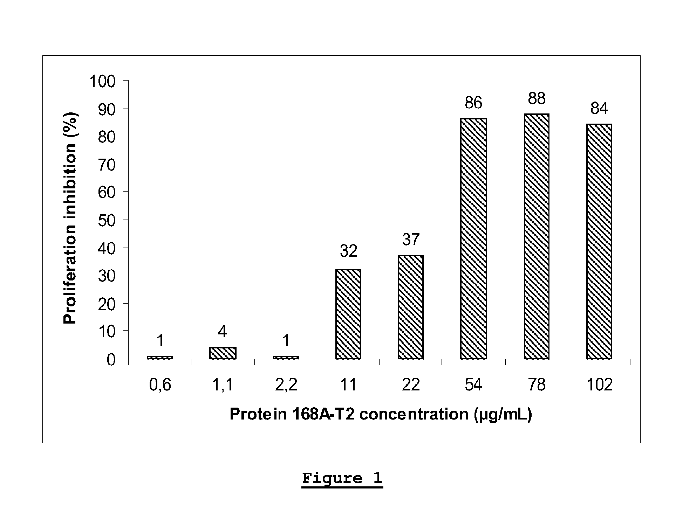

As shown in Figure 1, protein 168A-T2 inhibited human endothelial cell proliferation in a dose dependent manner. This inhibition represented 80% at 54µg/mL (i.e. 3.1µM) of protein 168A-T2.

### EXAMPLE 3: Inhibition of in vitro angiogenesis by 168A-T2

The purified proteins 168A-T2 was tested *in vitro* on angiogenesis of HUVEC induced by FGF2 and VEGF on Matrigel.

24 wells plates were prepared with 250µL of BD Matrigel™/well and then incubated 30 minutes in incubator. HUVEC cells were then prepared as described in example 2 and 70 000 cells (in 0.5mL) were seeded per well and incubated with different concentrations of the purified protein 168A-T2, in Tris-HCl 20 mM buffer (pH 8), containing 150mM NaCl and urea 2M; this buffer was used as control:
- Figure 2a: Control (Buffer Urea 2M)
- Figure 2b: 168A-T2 3.5µg/mL (0.2µM)
- Figure 2c: 168A-T2 6.9µg/mL (0.4µM)
- Figure 2d : 168A-T2 13.6µg/mL (0.8µM)

As shown in Figures 2b, 2c, and 2d, protein 168AT2 inhibited *in vitro* angiogenesis in a dose-dependent manner.

### EXAMPLE 4: Inhibition of the migration of human endothelial cells by 168A-T2

Cell migration was tested by the wound assay described by Sato and Rifkin (J Cell Biol. 1988;107:1199) with few modifications. HUVEC grown in growth medium EGM-2MV (Cambrex) were seeded in 24-well plates at 80 000 cells per well in 500µL of growth medium and grown to confluence at 37°C in a humidified atmosphere containing 5% CO₂. Cells were scrapped with a plastic tip on one line only. After wounding, the culture medium was changed for fresh medium (control, figure 3a) or fresh medium supplemented with:
- Figure 3b: 168A-T2 12µg/mL (0.7µM)
- Figure 3c: 168A-T2 17µg/mL (1µm)
- Figure 3d: 168A-T3 23µg/mL (1.35µM)

After 18 hours of culture, cells were observed and photographed under the inverted microscope (Analysis, Olympus, Rungis, France).

As shown in Figures 3b, 3c and 3d, protein 168A-T2 inhibited human endothelial cells migration in a dose dependent manner.

### EXAMPLE 5: Test of inhibition of a kidney cancer cell line proliferation by 168A-T2 in vitro

BizX cell preparation of 50000 cells/mL was prepared in complete medium. In a 96 wells plate, 100 mL of the cell preparation was distributed in each well and then incubated with different concentrations of 168A-T2 (each concentration was tested in triplicate). After 48 hours of incubation at 37°C, 10 mL of MTT (5mg/L in water) were added in each well. After 3 hours of incubation at 37°C, the culture medium was eliminated and 100 mL of DMSO were added to solubilize MMT crystals. The optical density (OD) at 570 nm was then measured using µQuant micro-plate reader (Bio-Tek Instrument gmbh, Colmar, France) coupled to the KC4 (Bio-Tek) software. The OD was corrected by subtracting blank-well OD values (the OD values obtained from wells without cells), and the inhibition of cell proliferation was measured relative to control (OD obtained from wells with untreated kidney tumor cells representing the maximal proliferative response, i.e.100%).

As shown in Figure 4, protein 168A-T2 inhibited up to 20% of proliferation of a kidney cancer line (BizX).

### EXAMPLE 6: Test of inhibition of a lung cancer cell line proliferation by 168A-T2 in vitro

Calu6 cells were cultured and treated as described in example 5.

As shown in Figure 5, protein 168A-T2 inhibited up to 40% of proliferation of a lung cancer line (Calu6).

### EXAMPLE 7: Expression of protein 168A in tumor samples

A number of different human tumor samples were screened for the expression of the gene 168A. For each pathological sample, the periphery of the tumor was separated from the core of the tumor, and the expression of the gene 168A in these two area was compared after mRNA extraction followed by RT-PCR.

### Kidney tumor samples

19 pathological biopsies from kidney tumors were analysed. In 11 out of 19 patients, the expression of 168A was much higher in the core than in the periphery of the tumor. In 8 patients out of 19 patients, the expression of 168A was much higher in the periphery of the tumor than in the core.

### Lung tumor samples

40 pathological biopsies from human lung tumors were analysed. In 23 out of 40 patients, the expression of 168A was much higher in the core than in the periphery of the tumor.

### Colon tumor samples

33 pathological biopsies from human colon tumors were analysed. In 13 out of 33 patients, the expression of 168A was much higher in the periphery than in the core of the tumor.

### EXAMPLE 8: Test of 168A-T2 on human non-small cell lung cancer (CALU-6) xenograft model in swiss nude mice in vivo Preparation of CALU-6 cells

CALU-6 cells were cultured as adherent cells in complete EMEM medium (Ref. CM1MEM18-01, batch No. 462502, Eurobio, France) 10% fetal calf serum (FCS; Ref. CVFSVF00-01, batch No. S13021, Eurobio, France) under a 37°C, 5% CO₂ humidified atmosphere. They were amplified in 75 cm²-flasks to reach 90x10⁶ cells.

At D0, CALU-6 cells (human lung carcinoma) were collected from 75 cm²-flasks by removing the medium and adding 3 ml of trypsine-EDTA (Ref. CEZTDA00-0U, batch No. 633920, Eurobio, France). After 5 min of incubation at 37°C, cells had detached from the plastic and the enzymatic reaction was stopped by adding 3 ml of EMEM medium containing 10% fetal calf serum. Cells were then centrifuged at 700 g for 5 min at room temperature. They were resuspended in serum-free EMEM culture medium. Cells were counted and viability assessment by Trypan Blue exclusion (Ref. CSTCOL03-OU, batch No. 434511, Eurobio, France). The number of viable CALU-6 cells was > 99%. The number of cells was then adjusted to 25x10⁶ cells/ml in serum-free medium.

### Tumor induction

Thirty healthy female Swiss *Nude* mice were anesthetized by IP injection of Ketamine-Xylazine (80 mg/kg-12 mg/kg; Ref. K-113, Sigma, France). CALU-6 cells 5x10⁶ cells/mouse in 200 µl of serum-free medium) were then implanted subcutaneously in the right flank of each mouse. Mice were observed for 2h post-implantation.

### Treatment schedule

At D12 post-implantation of the CALU-6 cells, the thirty mice were randomized into four groups of 5 mice. Tumor volumes had reached 54 to 296 mm³ and mean tumor volumes were not statistically different between groups after randomization.
The treatment schedule, starting D12 and ending D28, is summarized in Table 2.
- Animals of group 1 were treated with the vehicle solution (Tris-HCl pH 7.5, 2M Urea, 150mM NaCl, 0.1mM CaCl₂) ;
- Animals of group 2 were treated with a solution of cisplatin in physiological serum at a concentration of 0.5 mg/mL (CDDP, cis-diamineplatinum(II) dichloride, Ref. P4394, batch No. 014K0993, Sigma, France, purity 100%, MW. 300),
- Animals of group 3 were treated with the vehicle supplemented with the test substance 168A-T2 at a dose of 15 mg/kg.
- Animals of group 4 were treated with the vehicle supplemented with the test substance 168A-T2 at a dose of 15 mg/kg, and further received 5 mg/kg of CDDP.

Injections in groups 1, 2, 3 and 4 were performed according to the schedules Q2DX8, i.e. 1 quantity every two days, eight times.

Mice were observed for 2 hours post-injection. Ketamine/Xylazine (80 mg/kg - 12 mg/kg; Ref. K-113, Sigma, France) was used to anaesthetize the animals before sacrifice by cervical dislocation. For all animals, the tumor size was measured twice a week with calipers. The tumor volume (mm³) was measured according to the formula: (length x width²)/2.

### Statistical studies

Data outlined below were calculated:
- Tumor growth curves were drawn using the mean tumor volumes (MTV),
- Mean Relative tumor volume (MRTV) was calculated as the ratio between the MTV at time t and the volume at the time of injection (t=D12),
- Tumor growth inhibition (T/C, %) was evaluated as the ratio of the median tumor volumes of treated groups *versus* vehicle group.

Statistical analyses of tumor volumes (TV), time to reach 'TV', tumor-doubling time (DT), relative tumor volume (RTV) and tumor growth inhibition (T/C) were performed for all groups. Data are expressed as mean±SD. Groups of data were normally distributed. Univariate analysis were performed to assess differences between groups. Statistical significance was then determined using the Student's t test. A P<0.05 was considered as statistically significant. The Statistical analysis was performed using XLSTAT (Addinsoft, France).

### Body weight

As shown in table 3, the vehicle had no impact: mouse behavior and body weight gain were normal and no animal died prematurely. No toxicity was observed during the course of the treatment with the test substance 168A-T2 at the dose of 15 mg/kg, a slight body weight gain was observed (+2,45g).

In contrast, an important toxicity was observed in groups 2, 4 treated with CDDP (-2,70g and -2,35g body weight loss respectively). The difference between group 1 versus 2 and 4 and group 3 versus 2 and 4 was statistically significant (p<0,0001) but the difference between group 2 and 4 was not statistically significant.

**Table 2**

| **Group** | **Animals n** | **Treatment** | **Administration route** | **Treatment dose (mg/kg/adm)** | **Administration volume** | **Treatment schedule** |
|---|---|---|---|---|---|---|
| 1 | 5 | Vehicle | IP | 0 | 10 ml/kg | Q2DX8 |
| 2 | 5 | Cisplatin | IP | 5 | | Q2DX8 |
| 3 | 5 | 168A-T2 | IP | 15 | | Q2DX8 |
| 4 | 5 | 168A-T2 & Cisplatin | IP | 15 5 | | Q2DX8 |

**Table 3: Mean body weight (MBW) of mice bearing CALU-6 tumors treated with the vehicle, CDDP at 5 mg/kg (schedule Q2DX8, G2), 168A-T2 at 10.0 mg/kg (schedule Q2DX8, G3), combined 168A-T2 at 10.0 mg/kg and CDDP at 5 mg/kg (schedule Q2DX8, G4) at D12 and D28.**

| **Group** | **Test substance** | **Treatment dose (mg/kg)** | **MBW at D12 (g)** | **MBW at D28 (g)** | **MBWC D12-D28 (g)** |
|---|---|---|---|---|---|
| 1 | Vehicle | 0 | 22.35 ± 1.17 | 24.40 ± 1.19 | +2,36 (± 0.59) |
| 2 | Cisplatin | 5.00 | 21.40 ± 0.85 | 18.69 ± 1.97 | -2.70 (± 1.57) |
| 3 | 168A-T2 | 15.0 | 21.74 ± 0.89 | 24.20 ± 1.37 | +2.45 (± 0.65) |
| 4 | 168A-T2+ Cisplatin | 15.0 + 5.00 | 20.74 ± 1.41 | 18.39 ± 0.74 | -2,35 (± 1.99) |

The results of mean tumor volume *(MTV),* mean relative tumor volume *(MRTV)* and tumor growth parameters (T/C) are shown in Figures 6, 7 and in Tables 4, 5 and 6.

The MTV (Table 4, Figure 6) was decreased at D28 in mice of group 2 treated with CDDP (742,44 mm³) compared to mice of the vehicle group 1 (1233,44 mm³). The MTV at D28 was also decreased in group 3 treated with the test substance 168A-T2 at 15 mg/kg with 1 injection per two days (615,96 mm³). The most important MTV decrease was obtained for animals of group 4 (317,17 mm³). The difference between group 1 and the 2 groups treated with the test substance reach the statistical significativity (p< 0,0001 vs 4 - p = 0,003 vs 3). The difference between group 2 and 4 was also significant (p=0,001). In contrast no statistical difference was observed between group 2 (CDDP alone) and group 3 (168A-T2 alone).

**Table 4: Mean tumor volume (MTV) of animals bearing CALU-6 cells and treated with vehicle (group 1), CDDP alone (Group 2), 168A-T2 (10.0 mg/kg) (Group 3), or combined with 168AT2 and CDDP (Group 4) according to the scheduled treatment Q2DX8.**

| **MTV (mm³)** | | | | | | | | | |
|---|---|---|---|---|---|---|---|---|---|
| **Group** | **D12** | **D14** | **D16** | **D18** | **D20** | **D22** | **D24** | **D26** | **D28** |
| **1** | 142,61 | 252,49 | 365,52 | 522,27 | 784,61 | 588,53 | 803,89 | 1044,02 | 1233,44 |
| **2** | 142,60 | 351,15 | 291,29 | 329,18 | 386,84 | 470,59 | 595,00 | 527,98 | 742,44 |
| **3** | 159,22 | 181,94 | 264,20 | 322,99 | 391,90 | 485,42 | 642,24 | 524,95 | 615,96 |
| **4** | 137,68 | 157,35 | 250,14 | 226,39 | 290,70 | 311,24 | 387,85 | 334,10 | 317,17 |

These results were confirmed by the analysis of the MRTV (table 5, Figure 7). The MRTV for animals of group 1 was 8,24 at D28. For animals of group 2, i.e. treated with CDDP, the MRTV at D28 was 5,98; for animals of group 3, i.e. treated with 168A-T2 (15mg/kg), the MRTV at D28 was 4,51; eventually, for animals of group 4, i.e. treated with both CDDP and 168A-T2, the MRTV was 2,18.

**Table 5: Mean Relative tumor volume (MRTV) of animals bearing CALU-6 cells and treated with vehicle (group 1), CDDP alone (Group 2), 168A-T2 (10.0 mg/kg) (Group 3), or combined with 168A-T2 and CDDP (Group 4) according to the scheduled treatment Q2DX8.**

| **MRTV** | | | | | | | | | |
|---|---|---|---|---|---|---|---|---|---|
| **Group** | **D12** | **D14** | **D16** | **D18** | **D20** | **D22** | **D24** | **D26** | **D28** |
| **1** | 1 | 1,83 | 3,17 | 2,51 | 3,68 | 4,16 | 5,68 | 7,49 | 8,24 |
| **2** | 1 | 3,11 | 2,39 | 2,66 | 3,08 | 3,70 | 4,84 | 4,11 | 5,98 |
| **3** | 1 | 1,15 | 1,93 | 2,08 | 2,60 | 3,47 | 4,83 | 4,14 | 4,51 |
| **4** | 1 | 1,09 | 1,80 | 1,70 | 2,10 | 2,28 | 2,72 | 2,33 | 2,18 |

As shown in table 5 and figure 7, MRTV reached 2,18 at D28 for the animals of group 4, which confirmed the synergistic efficacy of 168A-T2 with Cisplatin. Moreover, cisplatin used alone (group 2) or 168A-T2 used alone (group 3) showed close MRTV at D28, suggesting that 168A-T2 is also a potent mono-therapy anti-tumor agent.

**Table 6: Growth inhibition based on T/C ratio**

| **T/C ratio (%)** | | | | | | | | |
|---|---|---|---|---|---|---|---|---|
| **Day** | **D14** | **D16** | **D18** | **D20** | **D22** | **D24** | **D26** | **D28** |
| **G2** | -70% | 24% | -6% | 17% | 11% | 15% | 45% | 27% |
| **G3** | 37% | 39% | 17% | 29% | 16% | 15% | 45% | 45% |
| **G4** | 40% | 43% | 32% | 43% | 45% | 52% | 69% | 74% |

The T/C ratio (table 6), which is a parameter of tumor growth inhibition, revealed a slight anti-tumoral activity of the test substance when used as a monotherapy as it reduced by 27% tumor size compared to the vehicle-treated group 1. However, when combined with CDDP, the inhibition rate reached 74% reduction of tumor size relative to the vehicle-treated group 1.

These results directly demonstrate that 168A-T2 has a potent anti-tumoral activity when it is used alone or in combination with a cytotoxic agent such as CDDP.

## Claims

1. A nucleic acid having the nucleic acid sequence of SEQ ID NO:3, or having at least 50%, preferably 70%, more preferably 90% identity with the nucleic acid sequence of SEQ ID NO:3, or fragments thereof having at least 60 contiguous nucleotides, or nucleic acid sequences having at least 50%, preferably 70%, more preferably 90% identity with the nucleic acid sequence of said fragments, provided that said nucleic acid is not SEQ ID NO:1.

2. An expression vector comprising at least one nucleic acid sequence according to claim **1.**

3. An active polypeptide comprising the amino acid sequence of SEQ ID NO:4, or having at least 50%, preferably 70%, more preferably 90% identity with the amino acid sequence of SEQ ID NO:4, or fragments thereof having at least 20 contiguous amino acids, or peptides having at least 50%, preferably 70%, more preferably 90% identity with the amino acid sequence of said fragments, provided that said polypeptide is not SEQ ID NO:2.

4. An active polypeptide according to claim **3, characterized in that** it is produced by the expression vector of claim **2.**

5. The polypeptide according to claim **3** or **4,** wherein said polypeptide has an anti-tumour activity.

6. A medicament comprising at least one nucleic acid sequence according to claim **1,** or at least one expression vector according to claim **2,** or at least one polypeptide according to anyone of claim **3** to **5.**

7. A pharmaceutical composition comprising at least one nucleic acid sequence according to claim **1,** or at least one expression vector according to claim **2,** or at least one polypeptide according to anyone of claim **3** to **5,** and one or more pharmaceutically acceptable excipients.

8. A pharmaceutical composition comprising at least one nucleic acid sequence according to claim **1,** or at least one expression vector according to claim **2,** or at least one polypeptide according to anyone of claim **3** to **5,** and one or more pharmaceutically acceptable excipients, for use in a method of treatment of cancer and/or tumours of the human or animal body.

9. A pharmaceutical composition according to claim **7** or **8,** further comprising at least one another active substance selected from anti-angiogenic substances or anti-tumour substances.

10. A pharmaceutical composition comprising effective amounts of
- a polypeptide according to anyone of claim **3** to **5,** and
- a platinum complex selected from the group consisting of cisplatin and carboplatin.

11. The pharmaceutical composition according to anyone of claims **7-10,** being in a form suitable for topical, systemic, oral, subcutaneous, transderm, intramuscular or intra-peritoneal administration.

12. The pharmaceutical composition according to anyone of claims **7-11,** wherein said polypeptide is present in an amount from 0.01 to 90% in weight, preferably from 0.1% to 10% in weight, more preferably from 1% to 5% in weight.

13. Use of the nucleic acid of claim **1,** or of the expression vector of claim **2,** or of the polypeptide of claim **3** to **5,** or of the medicament of claim **6,** or of the pharmaceutical composition of claims **7-12,** for the treatment of cancers and/or tumours.

14. The use according to claim **13,** wherein the tumours are solid tumours.

15. The use according to claim **14**, wherein the solid tumours are selected from sarcomas, carcinomas, and lymphomas.


SEQUENCE LISTING

<110>  GENE SIGNAL INTERNATIONAL SA

<120>  Anti-tumor drug, medicament, composition, and use thereof

<130>  BET070029

<160>  20

<170>  PatentIn version 3.3

<210>  1
<211>  6160
<212>  DNA
<213>  Homo sapiens

<400>  1
ctcgcgagga gagcggagca ggcgcgcggc ccaggcggag gagcgccgac tctggagcag      60

ccggagctgg aagaggagga ggaggagagg cggcggggaa ggaggaggag ggggagagtc     120

gctcccgccg ggcgagcatg gggcgcctgg cctcgaggcc gctgctgctg gcgctcctgt     180

cgttggctct ttgccgaggg cgtgtggtga gagtccccac agcgaccctg gttcgagtgg     240

tgggcactga gctggtcatc ccctgcaacg tcagtgacta tgatggcccc agcgagcaaa     300

actttgactg gagcttctca tctttgggga gcagctttgt ggagcttgca agcacctggg     360

aggtggggtt cccagcccag ctgtaccagg agcggctgca gaggggcgag atcctgttaa     420

ggcggactgc caacgacgcc gtggagctcc acataaagaa cgtccagcct tcagaccaag     480

gccactacaa atgttcaacc cccagcacag atgccactgt ccagggaaac tatgaggaca     540

cagtgcaggt taaagtgctg gccgactccc tgcacgtggg ccccagcgcg cggcccccgc     600

cgagcctgag cctgcgggag ggggagccct tcgagctgcg ctgcactgcc gcctccgcct     660

cgccgctgca cacgcacctg gcgctgctgt gggaggtgca ccgcggcccg gccaggcgga     720

gcgtcctcgc cctgacccac gagggcaggt tccacccggg cctggggtac gagcagcgct     780

accacagtgg ggacgtgcgc ctcgacaccg tgggcagcga cgcctaccgc ctctcagtgt     840

cccgggctct gtctgccgac cagggctcct acaggtgtat cgtcagcgag tggatcgccg     900

agcagggcaa ctggcaggaa atccaagaaa aggccgtgga agttgccacc gtggtgatcc     960

agccatcagt tctgcgagca gctgtgccca agaatgtgtc tgtggctgaa ggaaaggaac    1020

tggacctgac ctgtaacatc acaacagacc gagccgatga cgtccggccc gaggtgacgt    1080

ggtccttcag caggatgcct gacagcaccc tacctggctc ccgcgtgttg gcgcggcttg    1140

accgtgattc cctggtgcac agctcgcctc atgttgcttt gagtcatgtg gatgcacgct    1200

cctaccattt actggttcgg gatgttagca aagaaaactc tggctactat tactgccacg    1260

tgtccctgtg ggcacccgga cacaacagga gctggcacaa agtggcagag gccgtgtctt    1320

ccccagctgg tgtgggtgtg acctggctag aaccagacta ccaggtgtac ctgaatgctt    1380

ccaaggtccc cgggtttgcg gatgacccca cagagctggc atgccgggtg gtggacacga    1440

agagtgggga ggcgaatgtc cgattcacgg tttcgtggta ctacaggatg aaccggcgca    1500

gcgacaatgt ggtgaccagc gagctgcttg cagtcatgga cggggactgg acgctaaaat 1560
atggagagag gagcaagcag cgggcccagg atggagactt tattttttct aaggaacata 1620
cagacacgtt caatttccgg atccaaagga ctacagagga agacagaggc aattattact 1680
gtgttgtgtc tgcctggacc aaacagcgga acaacagctg ggtgaaaagc aaggatgtct 1740
tctccaagcc tgttaacata ttttgggcat tagaagattc cgtgcttgtg gtgaaggcga 1800
ggcagccaaa gcctttcttt gctgccggaa atacatttga gatgacttgc aaagtatctt 1860
ccaagaatat taagtcgcca cgctactctg ttctcatcat ggctgagaag cctgtcggcg 1920
acctctccag tcccaatgaa acgaagtaca tcatctctct ggaccaggat tctgtggtga 1980
agctggagaa ttggacagat gcatcacggg tggatggcgt tgttttagaa aaagtgcagg 2040
aggatgagtt ccgctatcga atgtaccaga ctcaggtctc agacgcaggg ctgtaccgct 2100
gcatggtgac agcctggtct cctgtcaggg gcagcctttg gcgagaagca gcaaccagtc 2160
tctccaatcc tattgagata gacttccaaa cctcaggtcc tatatttaat gcttctgtgc 2220
attcagacac accatcagta attcggggag atctgatcaa attgttctgt atcatcactg 2280
tcgagggagc agcactggat ccagatgaca tggcctttga tgtgtcctgg tttgcggtgc 2340
actcttttgg cctggacaag gctcctgtgc tcctgtcttc cctggatcgg aagggcatcg 2400
tgaccacctc ccggagggac tggaagagcg acctcagcct ggagcgcgtg agtgtgctgg 2460
aattcttgct gcaagtgcat ggctccgagg accaggactt tggcaactac tactgttccg 2520
tgactccatg ggtgaagtca ccaacaggtt cctggcagaa ggaggcagag atccactcca 2580
agcccgtttt tataactgtg aagatggatg tgctgaacgc cttcaagtat cccttgctga 2640
tcggcgtcgg tctgtccacg gtcatcgggc tcctgtcctg tctcatcggg tactgcagct 2700
cccactggtg ttgtaagaag gaggttcagg agacacggcg cgagcgccgc aggctcatgt 2760
cgatggagat ggactaggct ggcccgggag gggagtgaca gagggacgtt ctaggagcaa 2820
ttggggcaag aagaggacag tgatatttta aaacaaagtg tgttacacta aaaaccagtc 2880
ctctctaatc tcaggtggga cttggcgctc tctcttttct gcatgtcaag ttctgagcgc 2940
ggacatgttt accagcacac ggctcttctt cccacggcac tttctgatgt aacaatcgag 3000
tgtgtgtttt cccaactgca gctttttaat ggttaacctt catctaattt tttttctccc 3060
actggtttat agatcctctg acttgtgtgt gtttatagct tttgtttcgc ggggttgtgg 3120
tgaggaaggg gtgatggcat gcggagttct ttatcttcag tgagaatgtg cctgcccgcc 3180
tgagagccag cttccgcgtt ggaggcacgt gttcagagag ctgctgagcg ccaccctcta 3240
cccggctgac agacaacaca gacctgtgcc gaaggctaat ttgtggcttt tacgacccta 3300
ccccaccccc tgttttcagg ggtttagact acatttgaaa tccaaacttg gagtatataa 3360
cttcttattg agcccaactg cttttttttt tttttttttt gcttctctgc cccttttcca 3420
tttcttttgt atttgttttc tgtgagagca ctgaaatggc agccctggaa tctacaattt 3480
ggctctccac tgagcacctt atcttgccac cttagcctta agaatgaata tgaagaaaaa 3540

tacacagcca cctctgtcca gggcagtaag aagggctgca aggaagggga ggatggggac 3600
aaggaaagga tcagatacct gctccagtag ttgtgaggcc actgtgtctc aggggactcc 3660
aggaggagca gaagagggat cccacgaagt tattcttacg cagctggggc caggagggtc 3720
agagtggtgc caggtgcaag ttaggctaaa gaagccacca ctattcctct ctcttgccca 3780
ttgtgggggg caaaggcatt ggtcaccaag agtcttgcag ggggacccac agatatgcca 3840
tgtccttcac acgtgcttgg gctccttaac ctgaaggcaa attgctactt gcaagactga 3900
ctgacttcaa ggaatcagaa attacctaga agcaccatgt tttttctatg accttttcag 3960
tccttcaggt cattttaagg tccactgcag ggggttagtg agaaagggta tactttgtgg 4020
tatgttttgc tttcctaata gggacatgaa ggaaacccag caatttgctg ttatgtgaat 4080
ggcctgtaga gcagagtcaa gagcggtgtg ctttgcccga ctgctcccat caggaatagg 4140
agagtagaca gagatcttcc acatcccagg cttctgctgc tgctttaaaa gctctgtcct 4200
tggagcctcc cgctccctga agtgtctcgc cccctgcaca gcactggcct ttcggaagca 4260
tcccagtagg gttttctgag gctcgctggt gactcatgcc ctaattgcaa tcctctgctt 4320
ttatcttgac tttgaaggat ctaacactgc tctctcttcc aaaggggaaa aaaagattca 4380
tttgttttga gcaataaact aatacaaaat gatggccatt catgtgcagc tctttgtcac 4440
catgggccgg atgagttgtg ctcctcctgg ctcaccattt ccccctgctc ccccacagcc 4500
ggttctgcac ttatcaccga gtcgcccctg gaagcagatt cccattgagt tttccccacc 4560
aaggggacca tgcacatggt agaaacatta gattctgcat tgacagtagc ctttccttgg 4620
cccgggcctg tggtgggaag acgggcaaca agtatacccc accagggcct gagtgactag 4680
aggaagagga cgaggccttg ttggcactag atttgggtat tttctgcatg tcataacata 4740
tcctaactgc tatttcagaa gaggcagctt gtaggtgatt gtacaagtga gaattaaaga 4800
gagaacagat atttaaacag gtgctgtatt agtaacagcc agtgcccttt cagcccttgc 4860
atctattaaa aggagattca ggattttatt ggcacaggcc cttcttagta ggaagaaagg 4920
gtgcttagct ttggacctga ccgggtgtgt gtaaaaccat ggactgagtc acagcagaca 4980
ctcgatggtg gtaaatgtga tgggtgctta cacactgtac cttttccttt catactgatg 5040
ctgcagttca gggctggagt tgttaaggca ttgacctcca cccacctgcc ccatgtccac 5100
tgggctgccc aagctgcatg tcacctgagg gctggcagga aggggcgaga aatcccaggg 5160
cattgtacca aggacctagt tccttctagg gatataaatt tccaggaatg tgtattttta 5220
atgtggtgag atgcactctt ttgttgtacc aaatagggct ccccacccca cccctgcgac 5280
aagtgctctt ctagaacagg ttcctaccag cagcactggt gtgaatgaaa gagagaccca 5340
gccgcgtctc acacaggtgg aattgcactt cttaacaaaa aggaacttta taaaagtttg 5400
ggattttttt tcctaatcat aaaaatagcc ccagaaagag cctaagctat gttcagatag 5460
aagcctcgaa attcctgtaa attgtttact ttatgatgtt tacatacacg tttcactttg 5520
aaaaaaaatg caaatcgact ttttaacaac tgttgagatg tttcatggga cagtagaact 5580

ctgactcacc aactgggcta aattttaatt taaaaatgta tttatttgag tgtctttccc 5640

cccctcaccc tcaccatctg aggggctccc tgagatcttg gtagaggagg cccctcctgc 5700

ccagaccttc gtttgtttcc ccggtggccc ttgcttcttg ctttgcagac tgcctgcagc 5760

catgattttg tcactgacat ctgtgagcca aagactgagc ctttttggca ggaataataa 5820

gcaatactac acaacttgct actttcagaa aacttttttt tagcttcacc gatgacaaca 5880

gaggaagaag ggaactggga tttgggtaag ttctcctcca ctgtttgacc aaattctcag 5940

tgataaatat gtgtgcagat ccctagaaga gaaaacgctg actttctttt taagtgtggc 6000

acataaggat ctgcagaatt ttccgtagac aaagaaagga tcttgtgtat ttttgtccat 6060

atccaatgtt atatgaacta attgtattgt tttatactgt gaccacaaat attatgcaat 6120

gcaccatttg ttttttattt cattaaagga agtttaattt 6160

<210> 2
<211> 924
<212> PRT
<213> Homo sapiens

<400> 2
   Arg Glu Glu Ser Gly Ala Gly Ala Arg Pro Arg Arg Arg Ser Ala Asp
   1 5 10 15
   Ser Gly Ala Ala Gly Ala Gly Arg Gly Gly Gly Gly Glu Ala Ala Gly
   20 25 30
   Lys Glu Glu Glu Gly Glu Ser Arg Ser Arg Arg Ala Ser Met Gly Arg
   35 40 45
   Leu Ala Ser Arg Pro Leu Leu Leu Ala Leu Leu Ser Leu Ala Leu Cys
   50 55 60
   Arg Gly Arg Val Val Arg Val Pro Thr Ala Thr Leu Val Arg Val Val
   65 70 75 80
   Gly Thr Glu Leu Val Ile Pro Cys Asn Val Ser Asp Tyr Asp Gly Pro
   85 90 95
   Ser Glu Gln Asn Phe Asp Trp Ser Phe Ser Ser Leu Gly Ser Ser Phe
   100 105 110
   Val Glu Leu Ala Ser Thr Trp Glu Val Gly Phe Pro Ala Gln Leu Tyr
   115 120 125
   Gln Glu Arg Leu Gln Arg Gly Glu Ile Leu Leu Arg Arg Thr Ala Asn
   130 135 140
   Asp Ala Val Glu Leu His Ile Lys Asn Val Gln Pro Ser Asp Gln Gly
   145 150 155 160
   His Tyr Lys Cys Ser Thr Pro Ser Thr Asp Ala Thr Val Gln Gly Asn
                   165                 170                 175
   Tyr Glu Asp Thr Val Gln Val Lys Val Leu Ala Asp Ser Leu His Val
               180                 185                 190
   Gly Pro Ser Ala Arg Pro Pro Pro Ser Leu Ser Leu Arg Glu Gly Glu
           195                 200                 205
   Pro Phe Glu Leu Arg Cys Thr Ala Ala Ser Ala Ser Pro Leu His Thr
       210                 215                 220
   His Leu Ala Leu Leu Trp Glu Val His Arg Gly Pro Ala Arg Arg Ser
   225                 230                 235                 240
   Val Leu Ala Leu Thr His Glu Gly Arg Phe His Pro Gly Leu Gly Tyr
                   245                 250                 255
   Glu Gln Arg Tyr His Ser Gly Asp Val Arg Leu Asp Thr Val Gly Ser
               260                 265                 270
   Asp Ala Tyr Arg Leu Ser Val Ser Arg Ala Leu Ser Ala Asp Gln Gly
           275                 280                 285
   Ser Tyr Arg Cys Ile Val Ser Glu Trp Ile Ala Glu Gln Gly Asn Trp
       290                 295                 300
   Gln Glu Ile Gln Glu Lys Ala Val Glu Val Ala Thr Val Val Ile Gln
   305                 310                 315                 320
   Pro Ser Val Leu Arg Ala Ala Val Pro Lys Asn Val Ser Val Ala Glu
                   325                 330                 335
   Gly Lys Glu Leu Asp Leu Thr Cys Asn Ile Thr Thr Asp Arg Ala Asp
               340                 345                 350
   Asp Val Arg Pro Glu Val Thr Trp Ser Phe Ser Arg Met Pro Asp Ser
           355                 360                 365
   Thr Leu Pro Gly Ser Arg Val Leu Ala Arg Leu Asp Arg Asp Ser Leu
       370                 375                 380
   Val His Ser Ser Pro His Val Ala Leu Ser His Val Asp Ala Arg Ser
   385                 390                 395                 400
   Tyr His Leu Leu Val Arg Asp Val Ser Lys Glu Asn Ser Gly Tyr Tyr
                   405                 410                 415
   Tyr Cys His Val Ser Leu Trp Ala Pro Gly His Asn Arg Ser Trp His
               420                 425                 430
   Lys Val Ala Glu Ala Val Ser Ser Pro Ala Gly Val Gly Val Thr Trp
           435                 440                 445
   Leu Glu Pro Asp Tyr Gln Val Tyr Leu Asn Ala Ser Lys Val Pro Gly
       450                 455                 460
   Phe Ala Asp Asp Pro Thr Glu Leu Ala Cys Arg Val Val Asp Thr Lys
   465                 470                 475                 480
   Ser Gly Glu Ala Asn Val Arg Phe Thr Val Ser Trp Tyr Tyr Arg Met
                   485                 490                 495
   Asn Arg Arg Ser Asp Asn Val Val Thr Ser Glu Leu Leu Ala Val Met
               500                 505                 510
   Asp Gly Asp Trp Thr Leu Lys Tyr Gly Glu Arg Ser Lys Gln Arg Ala
           515                 520                 525
   Gln Asp Gly Asp Phe Ile Phe Ser Lys Glu His Thr Asp Thr Phe Asn
       530                 535                 540
   Phe Arg Ile Gln Arg Thr Thr Glu Glu Asp Arg Gly Asn Tyr Tyr Cys
   545                 550                 555                 560
   Val Val Ser Ala Trp Thr Lys Gln Arg Asn Asn Ser Trp Val Lys Ser
                   565                 570                 575
   Lys Asp Val Phe Ser Lys Pro Val Asn Ile Phe Trp Ala Leu Glu Asp
               580                 585                 590
   Ser Val Leu Val Val Lys Ala Arg Gln Pro Lys Pro Phe Phe Ala Ala
           595                 600                 605
   Gly Asn Thr Phe Glu Met Thr Cys Lys Val Ser Ser Lys Asn Ile Lys
       610                 615                 620
   Ser Pro Arg Tyr Ser Val Leu Ile Met Ala Glu Lys Pro Val Gly Asp
   625                 630                 635                 640
   Leu Ser Ser Pro Asn Glu Thr Lys Tyr Ile Ile Ser Leu Asp Gln Asp
                   645                 650                 655
   Ser Val Val Lys Leu Glu Asn Trp Thr Asp Ala Ser Arg Val Asp Gly
               660                 665                 670
   Val Val Leu Glu Lys Val Gln Glu Asp Glu Phe Arg Tyr Arg Met Tyr
           675                 680                 685
   Gln Thr Gln Val Ser Asp Ala Gly Leu Tyr Arg Cys Met Val Thr Ala
       690                 695                 700
   Trp Ser Pro Val Arg Gly Ser Leu Trp Arg Glu Ala Ala Thr Ser Leu
   705                 710                 715                 720
   Ser Asn Pro Ile Glu Ile Asp Phe Gln Thr Ser Gly Pro Ile Phe Asn
                   725                 730                 735
   Ala Ser Val His Ser Asp Thr Pro Ser Val Ile Arg Gly Asp Leu Ile
               740                 745                 750
   Lys Leu Phe Cys Ile Ile Thr Val Glu Gly Ala Ala Leu Asp Pro Asp
           755                 760                 765
   Asp Met Ala Phe Asp Val Ser Trp Phe Ala Val His Ser Phe Gly Leu
       770                 775                 780
   Asp Lys Ala Pro Val Leu Leu Ser Ser Leu Asp Arg Lys Gly Ile Val
   785                 790                 795                 800
   Thr Thr Ser Arg Arg Asp Trp Lys Ser Asp Leu Ser Leu Glu Arg Val
                   805                 810                 815
   Ser Val Leu Glu Phe Leu Leu Gln Val His Gly Ser Glu Asp Gln Asp
               820                 825                 830
   Phe Gly Asn Tyr Tyr Cys Ser Val Thr Pro Trp Val Lys Ser Pro Thr
           835                 840                 845
   Gly Ser Trp Gln Lys Glu Ala Glu Ile His Ser Lys Pro Val Phe Ile
       850                 855                 860
   Thr Val Lys Met Asp Val Leu Asn Ala Phe Lys Tyr Pro Leu Leu Ile
   865                 870                 875                 880
   Gly Val Gly Leu Ser Thr Val Ile Gly Leu Leu Ser Cys Leu Ile Gly
                   885                 890                 895
   Tyr Cys Ser Ser His Trp Cys Cys Lys Lys Glu Val Gln Glu Thr Arg
               900                 905                 910
   Arg Glu Arg Arg Arg Leu Met Ser Met Glu Met Asp
           915                 920

<210>  3
<211>  327
<212>  DNA
<213>  Homo sapiens

<400>  3
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaacta ctactgttcc gtgactccat gggtgaagtc accaacaggt     240

tcctggcaga aggaggcaga gatccactcc aagcccgttt ttataactgt gaagatggat     300

gtgctgaacg ccttcaagta tccctga                                         327

<210>  4
<211>  108
<212>  PRT
<213>  Homo sapiens

<400>  4

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn Tyr Tyr Cys Ser Val Thr Pro Trp Val Lys Ser Pro Thr Gly
65                  70                  75                  80

Ser Trp Gln Lys Glu Ala Glu Ile His Ser Lys Pro Val Phe Ile Thr
                85                  90                  95

Val Lys Met Asp Val Leu Asn Ala Phe Lys Tyr Pro
            100                 105

<210>  5
<211>  1049
<212>  DNA
<213>  Homo sapiens

<220>
<221>  misc_feature
<222>  (960)..(960)
<223>  n is a, c, g, or t

<220>
<221>  misc_feature
<222>  (963)..(963)
<223>  n is a, c, g, or t

<220>
<221>  misc_feature
<222>  (977)..(978)
<223>  n is a, c, g, or t

<220>
<221>  misc_feature
<222>  (981)..(981)
<223>  n is a, c, g, or t

<220>
<221> misc_feature
<222> (994)..(996)
<223> n is a, c, g, or t

<220>
<221> misc_feature
<222> (998)..(998)
<223> n is a, c, g, or t

<220>
<221> misc_feature
<222> (1012)..(1013)
<223> n is a, c, g, or t

<220>
<221> misc_feature
<222> (1019)..(1019)
<223> n is a, c, g, or t

<220>
<221> misc_feature
<222> (1023)..(1023)
<223> n is a, c, g, or t

<220>
<221> misc_feature
<222> (1029)..(1029)
<223> n is a, c, g, or t

<220>
<221> misc_feature
<222> (1036)..(1036)
<223> n is a, c, g, or t

<220>
<221> misc_feature
<222> (1047)..(1047)
<223> n is a, c, g, or t

<400> 5
ttcccctcta gaaataattt tgtttaactt taagaaggag atatacatat gcaccatcat 60

catcatcatt cttctggtct ggtgccacgc ggttctggta tgaaagaaac cgctgctgct 120

aaattcgaac gccagcacat ggacagccca gatctgggta ccgatgacga cgacaagatg 180

gcctttgatg tgtcctggtt tgcggtgcac tcttttggcc tggacaaggc tcctgtgctc 240

ctgtcttccc tggatcggaa gggcatcgtg accacctccc ggagggactg gaagagcgac 300

ctcagcctgg agcgcgtgag tgtgctggaa ttcttgctgc aagtgcatgg ctccgaggac 360

caggactttg gcaactacta ctgttccgtg actccatggg tgaagtcacc aacaggttcc 420

tggcagaagg aggcagagat ccactccaag cccgttttta taactgtgaa gatggatgtg 480

ctgaacgcct tcaagtatcc ctgaaccggg cttctcctca accatggcga tatcggatcc 540

gaattcgagc tccgtcgaca agcttgcggc cgcactcgag caccaccacc accaccactg 600

agatccggct gctaacaaag cccgaaagga agctgagttg gctgctgcca ccgctgagca 660

ataactagca taaccccttg gggcctctaa acgggtcttg aggggttttt tgctgaaagg 720

aggaactata tccggattgg cgaatgggac gcgccctgta gcggcgcatt aagcgcggcg 780

ggtgtggtgg ttacgcgcag cgtgaccgct acacttgcca gcgccctagc gcccgctctt 840

tcgctttctt ccttcctttc tcgccacgtt cgccggcttt ccccgtcaag ctctaaatcg 900

gggctccctt tagggttccg atttagtgct tacggccctc gaccccaaaa acttgattan 960

ggngatggtt cacgtanngg ncatcccctg atannngntt ttcgcctttg anntggagnc 1020

ccnttcttna tagggnacct tgttccnaa 1049

<210> 6
<211> 151
<212> PRT
<213> Homo sapiens

<400> 6

Met His His His His His His Ser Ser Gly Leu Val Pro Arg Gly Ser
1               5                   10                  15

Gly Met Lys Glu Thr Ala Ala Ala Lys Phe Glu Arg Gln His Met Asp
            20                  25                  30

Ser Pro Asp Leu Gly Thr Asp Asp Asp Asp Lys Met Ala Phe Asp Val
        35                  40                  45

Ser Trp Phe Ala Val His Ser Phe Gly Leu Asp Lys Ala Pro Val Leu
    50                  55                  60

Leu Ser Ser Leu Asp Arg Lys Gly Ile Val Thr Thr Ser Arg Arg Asp
65                  70                  75                  80

Trp Lys Ser Asp Leu Ser Leu Glu Arg Val Ser Val Leu Glu Phe Leu
                85                  90                  95

Leu Gln Val His Gly Ser Glu Asp Gln Asp Phe Gly Asn Tyr Tyr Cys
            100                 105                 110

Ser Val Thr Pro Trp Val Lys Ser Pro Thr Gly Ser Trp Gln Lys Glu
        115                 120                 125

Ala Glu Ile His Ser Lys Pro Val Phe Ile Thr Val Lys Met Asp Val
    130                 135                 140

Leu Asn Ala Phe Lys Tyr Pro
145                 150

<210> 7
<211> 90
<212> PRT
<213> Homo sapiens

<400> 7

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn Tyr Tyr Cys Ser Val Thr Pro Trp Val Lys Ser Pro Thr Gly
65                  70                  75                  80

Ser Trp Gln Lys Glu Ala Glu Ile His Ser
                85                  90

<210>  8
<211>  77
<212>  PRT
<213>  Homo sapiens

<400>  8

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn Tyr Tyr Cys Ser Val Thr Pro Trp Val Lys Ser
65                  70                  75

<210>  9
<211>  66
<212>  PRT
<213>  Homo sapiens

<400>  9

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn
65

<210> 10
<211> 51
<212> PRT
<213> Homo sapiens

<400> 10

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu
    50

<210> 11
<211> 37
<212> PRT
<213> Homo sapiens

<400> 11

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp
        35

<210> 12
<211> 20
<212> PRT
<213> Homo sapiens

<400> 12

Met Ala Phe Asp Val Ser Trp Phe Ala Val His Ser Phe Gly Leu Asp
1               5                   10                  15

Lys Ala Pro Val
            20

<210> 13
<211> 270
<212> DNA
<213> Homo sapiens

<400>  13
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaacta ctactgttcc gtgactccat gggtgaagtc accaacaggt     240

tcctggcaga aggaggcaga gatccactcc                                      270


<210>  14
<211>  231
<212>  DNA
<213>  Homo sapiens

<400>  14
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaacta ctactgttcc gtgactccat gggtgaagtc a              231


<210>  15
<211>  198
<212>  DNA
<213>  Homo sapiens

<400>  15
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaac                                                   198


<210>  16
<211>  153
<212>  DNA
<213>  Homo sapiens

<400>  16
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaa                                  153


<210>  17
<211>  111
<212>  DNA
<213>  Homo sapiens

<400>  17
atggcctttg atgtgtcctg gtttgcggtg cactcttttg gcctggacaa ggctcctgtg      60

ctcctgtctt ccctggatcg gaagggcatc gtgaccacct cccggaggga c              111


<210>  18
<211>  60

<212>  DNA
<213>  Homo sapiens

<400>  18
atggcctttg atgtgtcctg gtttgcggtg cactcttttg gcctggacaa ggctcctgtg      60


<210>  19
<211>  37
<212>  DNA
<213>  Homo sapiens

<400>  19
gacgacgaca agatggcctt tgatgtgtcc tggtttg                               37


<210>  20
<211>  43
<212>  DNA
<213>  Homo sapiens

<400>  20
gaggagaagc ccggttcagg gatacttgaa ggcgttcagc aca                        43

SEQUENCE LISTING

<110>  GENE SIGNAL INTERNATIONAL SA

<120>  Anti-tumor drug, medicament, composition, and use thereof

<130>  BET070029

<160>  18

<170>  PatentIn version 3.3

<210>  1
<211>  6160
<212>  DNA
<213>  Homo sapiens

<400>  1
ctcgcgagga gagcggagca ggcgcgcggc ccaggcggag gagcgccgac tctggagcag      60

ccggagctgg aagaggagga ggaggagagg cggcggggaa ggaggaggag ggggagagtc     120

gctcccgccg ggcgagcatg gggcgcctgg cctcgaggcc gctgctgctg gcgctcctgt     180

cgttggctct ttgccgaggg cgtgtggtga gagtccccac agcgaccctg gttcgagtgg     240

tgggcactga gctggtcatc ccctgcaacg tcagtgacta tgatggcccc agcgagcaaa     300

actttgactg gagcttctca tctttgggga gcagctttgt ggagcttgca agcacctggg     360

aggtggggtt cccagcccag ctgtaccagg agcggctgca gaggggcgag atcctgttaa     420

ggcggactgc caacgacgcc gtggagctcc acataaagaa cgtccagcct tcagaccaag     480

gccactacaa atgttcaacc cccagcacag atgccactgt ccagggaaac tatgaggaca     540

cagtgcaggt taaagtgctg gccgactccc tgcacgtggg ccccagcgcg cggcccccgc     600

cgagcctgag cctgcgggag ggggagccct tcgagctgcg ctgcactgcc gcctccgcct     660

cgccgctgca cacgcacctg gcgctgctgt gggaggtgca ccgcggcccg gccaggcgga     720

gcgtcctcgc cctgacccac gagggcaggt tccacccggg cctggggtac gagcagcgct     780

accacagtgg ggacgtgcgc ctcgacaccg tgggcagcga cgcctaccgc ctctcagtgt     840

cccgggctct gtctgccgac cagggctcct acaggtgtat cgtcagcgag tggatcgccg     900

agcagggcaa ctggcaggaa atccaagaaa aggccgtgga agttgccacc gtggtgatcc     960

agccatcagt tctgcgagca gctgtgccca agaatgtgtc tgtggctgaa ggaaaggaac    1020

tggacctgac ctgtaacatc acaacagacc gagccgatga cgtccggccc gaggtgacgt    1080

ggtccttcag caggatgcct gacagcaccc tacctggctc ccgcgtgttg gcgcggcttg    1140

accgtgattc cctggtgcac agctcgcctc atgttgcttt gagtcatgtg gatgcacgct    1200

cctaccattt actggttcgg gatgttagca aagaaaactc tggctactat tactgccacg    1260

tgtccctgtg ggcacccgga cacaacagga gctggcacaa agtggcagag gccgtgtctt    1320

ccccagctgg tgtgggtgtg acctggctag aaccagacta ccaggtgtac ctgaatgctt    1380

ccaaggtccc cgggtttgcg gatgacccca cagagctggc atgccgggtg gtggacacga    1440

agagtgggga ggcgaatgtc cgattcacgg tttcgtggta ctacaggatg aaccggcgca    1500

gcgacaatgt ggtgaccagc gagctgcttg cagtcatgga cggggactgg acgctaaaat    1560

atggagagag gagcaagcag cgggcccagg atggagactt tattttttct aaggaacata 1620
cagacacgtt caatttccgg atccaaagga ctacagagga agacagaggc aattattact 1680
gtgttgtgtc tgcctggacc aaacagcgga acaacagctg ggtgaaaagc aaggatgtct 1740
tctccaagcc tgttaacata ttttgggcat tagaagattc cgtgcttgtg gtgaaggcga 1800
ggcagccaaa gcctttcttt gctgccggaa atacatttga gatgacttgc aaagtatctt 1860
ccaagaatat taagtcgcca cgctactctg ttctcatcat ggctgagaag cctgtcggcg 1920
acctctccag tcccaatgaa acgaagtaca tcatctctct ggaccaggat tctgtggtga 1980
agctggagaa ttggacagat gcatcacggg tggatggcgt tgttttagaa aaagtgcagg 2040
aggatgagtt ccgctatcga atgtaccaga ctcaggtctc agacgcaggg ctgtaccgct 2100
gcatggtgac agcctggtct cctgtcaggg gcagcctttg gcgagaagca gcaaccagtc 2160
tctccaatcc tattgagata gacttccaaa cctcaggtcc tatatttaat gcttctgtgc 2220
attcagacac accatcagta attcggggag atctgatcaa attgttctgt atcatcactg 2280
tcgagggagc agcactggat ccagatgaca tggcctttga tgtgtcctgg tttgcggtgc 2340
actcttttgg cctggacaag gctcctgtgc tcctgtcttc cctggatcgg aagggcatcg 2400
tgaccacctc ccggagggac tggaagagcg acctcagcct ggagcgcgtg agtgtgctgg 2460
aattcttgct gcaagtgcat ggctccgagg accaggactt tggcaactac tactgttccg 2520
tgactccatg ggtgaagtca ccaacaggtt cctggcagaa ggaggcagag atccactcca 2580
agcccgtttt tataactgtg aagatggatg tgctgaacgc cttcaagtat cccttgctga 2640
tcggcgtcgg tctgtccacg gtcatcgggc tcctgtcctg tctcatcggg tactgcagct 2700
cccactggtg ttgtaagaag gaggttcagg agacacggcg cgagcgccgc aggctcatgt 2760
cgatggagat ggactaggct ggcccgggag gggagtgaca gagggacgtt ctaggagcaa 2820
ttggggcaag aagaggacag tgatatttta aaacaaagtg tgttacacta aaaaccagtc 2880
ctctctaatc tcaggtggga cttggcgctc tctcttttct gcatgtcaag ttctgagcgc 2940
ggacatgttt accagcacac ggctcttctt cccacggcac tttctgatgt aacaatcgag 3000
tgtgtgtttt cccaactgca gctttttaat ggttaacctt catctaattt tttttctccc 3060
actggtttat agatcctctg acttgtgtgt gtttatagct tttgtttcgc ggggttgtgg 3120
tgaggaaggg gtgatggcat gcggagttct ttatcttcag tgagaatgtg cctgcccgcc 3180
tgagagccag cttccgcgtt ggaggcacgt gttcagagag ctgctgagcg ccaccctcta 3240
cccggctgac agacaacaca gacctgtgcc gaaggctaat ttgtggcttt tacgacccta 3300
ccccaccccc tgttttcagg ggtttagact acatttgaaa tccaaacttg gagtatataa 3360
cttcttattg agcccaactg cttttttttt tttttttttt gcttctctgc cccttttcca 3420
tttcttttgt atttgttttc tgtgagagca ctgaaatggc agccctggaa tctacaattt 3480
ggctctccac tgagcacctt atcttgccac cttagcctta agaatgaata tgaagaaaaa 3540
tacacagcca cctctgtcca gggcagtaag aagggctgca aggaagggga ggatggggac 3600

aaggaaagga tcagatacct gctccagtag ttgtgaggcc actgtgtctc aggggactcc 3660
aggaggagca gaagagggat cccacgaagt tattcttacg cagctggggc caggagggtc 3720
agagtggtgc caggtgcaag ttaggctaaa gaagccacca ctattcctct ctcttgccca 3780
ttgtgggggg caaaggcatt ggtcaccaag agtcttgcag ggggacccac agatatgcca 3840
tgtccttcac acgtgcttgg gctccttaac ctgaaggcaa attgctactt gcaagactga 3900
ctgacttcaa ggaatcagaa attacctaga agcaccatgt tttttctatg accttttcag 3960
tccttcaggt cattttaagg tccactgcag ggggttagtg agaaagggta tactttgtgg 4020
tatgttttgc tttcctaata gggacatgaa ggaaacccag caatttgctg ttatgtgaat 4080
ggcctgtaga gcagagtcaa gagcggtgtg ctttgcccga ctgctcccat caggaatagg 4140
agagtagaca gagatcttcc acatcccagg cttctgctgc tgctttaaaa gctctgtcct 4200
tggagcctcc cgctccctga agtgtctcgc cccctgcaca gcactggcct ttcggaagca 4260
tcccagtagg gttttctgag gctcgctggt gactcatgcc ctaattgcaa tcctctgctt 4320
ttatcttgac tttgaaggat ctaacactgc tctctcttcc aaaggggaaa aaaagattca 4380
tttgttttga gcaataaact aatacaaaat gatggccatt catgtgcagc tctttgtcac 4440
catgggccgg atgagttgtg ctcctcctgg ctcaccattt ccccctgctc ccccacagcc 4500
ggttctgcac ttatcaccga gtcgcccctg gaagcagatt cccattgagt tttccccacc 4560
aaggggacca tgcacatggt agaaacatta gattctgcat tgacagtagc ctttccttgg 4620
cccgggcctg tggtgggaag acgggcaaca agtatacccc accagggcct gagtgactag 4680
aggaagagga cgaggccttg ttggcactag atttgggtat tttctgcatg tcataacata 4740
tcctaactgc tatttcagaa gaggcagctt gtaggtgatt gtacaagtga gaattaaaga 4800
gagaacagat atttaaacag gtgctgtatt agtaacagcc agtgcccttt cagcccttgc 4860
atctattaaa aggagattca ggattttatt ggcacaggcc cttcttagta ggaagaaagg 4920
gtgcttagct ttggacctga ccgggtgtgt gtaaaaccat ggactgagtc acagcagaca 4980
ctcgatggtg gtaaatgtga tgggtgctta cacactgtac cttttccttt catactgatg 5040
ctgcagttca gggctggagt tgttaaggca ttgacctcca cccacctgcc ccatgtccac 5100
tgggctgccc aagctgcatg tcacctgagg gctggcagga aggggcgaga aatcccaggg 5160
cattgtacca aggacctagt tccttctagg gatataaatt tccaggaatg tgtattttta 5220
atgtggtgag atgcactctt ttgttgtacc aaatagggct ccccacccca cccctgcgac 5280
aagtgctctt ctagaacagg ttcctaccag cagcactggt gtgaatgaaa gagagaccca 5340
gccgcgtctc acacaggtgg aattgcactt cttaacaaaa aggaacttta taaaagtttg 5400
ggattttttt tcctaatcat aaaaatagcc ccagaaagag cctaagctat gttcagatag 5460
aagcctcgaa attcctgtaa attgtttact ttatgatgtt tacatacacg tttcactttg 5520
aaaaaaaatg caaatcgact ttttaacaac tgttgagatg tttcatggga cagtagaact 5580
ctgactcacc aactgggcta aattttaatt taaaaatgta tttatttgag tgtctttccc 5640
cccctcaccc tcaccatctg aggggctccc tgagatcttg gtagaggagg cccctcctgc 5700

ccagaccttc gtttgtttcc ccggtggccc ttgcttcttg ctttgcagac tgcctgcagc 5760

catgattttg tcactgacat ctgtgagcca aagactgagc ctttttggca ggaataataa 5820

gcaatactac acaacttgct actttcagaa aacttttttt tagcttcacc gatgacaaca 5880

gaggaagaag ggaactggga tttgggtaag ttctcctcca ctgtttgacc aaattctcag 5940

tgataaatat gtgtgcagat ccctagaaga gaaaacgctg actttctttt taagtgtggc 6000

acataaggat ctgcagaatt ttccgtagac aaagaaagga tcttgtgtat ttttgtccat 6060

atccaatgtt atatgaacta attgtattgt tttatactgt gaccacaaat attatgcaat 6120

gcaccatttg tttttatttt cattaaagga agtttaattt 6160

<210> 2
<211> 924
<212> PRT
<213> Homo sapiens

<400> 2
   Arg Glu Glu Ser Gly Ala Gly Ala Arg Pro Arg Arg Arg Ser Ala Asp
   1 5 10 15
   Ser Gly Ala Ala Gly Ala Gly Arg Gly Gly Gly Gly Glu Ala Ala Gly
   20 25 30
   Lys Glu Glu Glu Gly Glu Ser Arg Ser Arg Arg Ala Ser Met Gly Arg
   35 40 45
   Leu Ala Ser Arg Pro Leu Leu Leu Ala Leu Leu Ser Leu Ala Leu Cys
   50 55 60
   Arg Gly Arg Val Val Arg Val Pro Thr Ala Thr Leu Val Arg Val Val
   65 70 75 80
   Gly Thr Glu Leu Val Ile Pro Cys Asn Val Ser Asp Tyr Asp Gly Pro
   85 90 95
   Ser Glu Gln Asn Phe Asp Trp Ser Phe Ser Ser Leu Gly Ser Ser Phe
   100 105 110
   Val Glu Leu Ala Ser Thr Trp Glu Val Gly Phe Pro Ala Gln Leu Tyr
   115 120 125
   Gln Glu Arg Leu Gln Arg Gly Glu Ile Leu Leu Arg Arg Thr Ala Asn
   130 135 140
   Asp Ala Val Glu Leu His Ile Lys Asn Val Gln Pro Ser Asp Gln Gly
   145 150 155 160
   His Tyr Lys Cys Ser Thr Pro Ser Thr Asp Ala Thr Val Gln Gly Asn
   165 170 175
   Tyr Glu Asp Thr Val Gln Val Lys Val Leu Ala Asp Ser Leu His Val
               180                 185                 190
   Gly Pro Ser Ala Arg Pro Pro Pro Ser Leu Ser Leu Arg Glu Gly Glu
           195                 200                 205
   Pro Phe Glu Leu Arg Cys Thr Ala Ala Ser Ala Ser Pro Leu His Thr
       210                 215                 220
   His Leu Ala Leu Leu Trp Glu Val His Arg Gly Pro Ala Arg Arg Ser
   225                 230                 235                 240
   Val Leu Ala Leu Thr His Glu Gly Arg Phe His Pro Gly Leu Gly Tyr
                   245                 250                 255
   Glu Gln Arg Tyr His Ser Gly Asp Val Arg Leu Asp Thr Val Gly Ser
               260                 265                 270
   Asp Ala Tyr Arg Leu Ser Val Ser Arg Ala Leu Ser Ala Asp Gln Gly
           275                 280                 285
   Ser Tyr Arg Cys Ile Val Ser Glu Trp Ile Ala Glu Gln Gly Asn Trp
       290                 295                 300
   Gln Glu Ile Gln Glu Lys Ala Val Glu Val Ala Thr Val Val Ile Gln
   305                 310                 315                 320
   Pro Ser Val Leu Arg Ala Ala Val Pro Lys Asn Val Ser Val Ala Glu
                   325                 330                 335
   Gly Lys Glu Leu Asp Leu Thr Cys Asn Ile Thr Thr Asp Arg Ala Asp
               340                 345                 350
   Asp Val Arg Pro Glu Val Thr Trp Ser Phe Ser Arg Met Pro Asp Ser
           355                 360                 365
   Thr Leu Pro Gly Ser Arg Val Leu Ala Arg Leu Asp Arg Asp Ser Leu
       370                 375                 380
   Val His Ser Ser Pro His Val Ala Leu Ser His Val Asp Ala Arg Ser
   385                 390                 395                 400
   Tyr His Leu Leu Val Arg Asp Val Ser Lys Glu Asn Ser Gly Tyr Tyr
                   405                 410                 415
   Tyr Cys His Val Ser Leu Trp Ala Pro Gly His Asn Arg Ser Trp His
               420                 425                 430
   Lys Val Ala Glu Ala Val Ser Ser Pro Ala Gly Val Gly Val Thr Trp
           435                 440                 445
   Leu Glu Pro Asp Tyr Gln Val Tyr Leu Asn Ala Ser Lys Val Pro Gly
   450                 455                 460
   Phe Ala Asp Asp Pro Thr Glu Leu Ala Cys Arg Val Val Asp Thr Lys
   465                 470                 475                 480
   Ser Gly Glu Ala Asn Val Arg Phe Thr Val Ser Trp Tyr Tyr Arg Met
                   485                 490                 495
   Asn Arg Arg Ser Asp Asn Val Val Thr Ser Glu Leu Leu Ala Val Met
               500                 505                 510
   Asp Gly Asp Trp Thr Leu Lys Tyr Gly Glu Arg Ser Lys Gln Arg Ala
           515                 520                 525
   Gln Asp Gly Asp Phe Ile Phe Ser Lys Glu His Thr Asp Thr Phe Asn
       530                 535                 540
   Phe Arg Ile Gln Arg Thr Thr Glu Glu Asp Arg Gly Asn Tyr Tyr Cys
   545                 550                 555                 560
   Val Val Ser Ala Trp Thr Lys Gln Arg Asn Asn Ser Trp Val Lys Ser
                   565                 570                 575
   Lys Asp Val Phe Ser Lys Pro Val Asn Ile Phe Trp Ala Leu Glu Asp
               580                 585                 590
   Ser Val Leu Val Val Lys Ala Arg Gln Pro Lys Pro Phe Phe Ala Ala
           595                 600                 605
   Gly Asn Thr Phe Glu Met Thr Cys Lys Val Ser Ser Lys Asn Ile Lys
       610                 615                 620
   Ser Pro Arg Tyr Ser Val Leu Ile Met Ala Glu Lys Pro Val Gly Asp
   625                 630                 635                 640
   Leu Ser Ser Pro Asn Glu Thr Lys Tyr Ile Ile Ser Leu Asp Gln Asp
                   645                 650                 655
   Ser Val Val Lys Leu Glu Asn Trp Thr Asp Ala Ser Arg Val Asp Gly
               660                 665                 670
   Val Val Leu Glu Lys Val Gln Glu Asp Glu Phe Arg Tyr Arg Met Tyr
           675                 680                 685
   Gln Thr Gln Val Ser Asp Ala Gly Leu Tyr Arg Cys Met Val Thr Ala
       690                 695                 700
   Trp Ser Pro Val Arg Gly Ser Leu Trp Arg Glu Ala Ala Thr Ser Leu
   705                 710                 715                 720
   Ser Asn Pro Ile Glu Ile Asp Phe Gln Thr Ser Gly Pro Ile Phe Asn
                   725                 730                 735
   Ala Ser Val His Ser Asp Thr Pro Ser Val Ile Arg Gly Asp Leu Ile
   740 745 750
   Lys Leu Phe Cys Ile Ile Thr Val Glu Gly Ala Ala Leu Asp Pro Asp
   755 760 765
   Asp Met Ala Phe Asp Val Ser Trp Phe Ala Val His Ser Phe Gly Leu
   770 775 780
   Asp Lys Ala Pro Val Leu Leu Ser Ser Leu Asp Arg Lys Gly Ile Val
   785 790 795 800
   Thr Thr Ser Arg Arg Asp Trp Lys Ser Asp Leu Ser Leu Glu Arg Val
   805 810 815
   Ser Val Leu Glu Phe Leu Leu Gln Val His Gly Ser Glu Asp Gln Asp
   820 825 830
   Phe Gly Asn Tyr Tyr Cys Ser Val Thr Pro Trp Val Lys Ser Pro Thr
   835 840 845
   Gly Ser Trp Gln Lys Glu Ala Glu Ile His Ser Lys Pro Val Phe Ile
   850 855 860
   Thr Val Lys Met Asp Val Leu Asn Ala Phe Lys Tyr Pro Leu Leu Ile
   865 870 875 880
   Gly Val Gly Leu Ser Thr Val Ile Gly Leu Leu Ser Cys Leu Ile Gly
   885 890 895
   Tyr Cys Ser Ser His Trp Cys Cys Lys Lys Glu Val Gln Glu Thr Arg
   900 905 910
   Arg Glu Arg Arg Arg Leu Met Ser Met Glu Met Asp
   915 920

<210>  3
<211>  327
<212>  DNA
<213>  Homo sapiens

<400>  3
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaacta ctactgttcc gtgactccat gggtgaagtc accaacaggt     240

tcctggcaga aggaggcaga gatccactcc aagcccgttt ttataactgt gaagatggat     300

gtgctgaacg ccttcaagta tccctga                                         327

<210>  4
<211>  108
<212>  PRT
<213>  Homo sapiens

<400>  4

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn Tyr Tyr Cys Ser Val Thr Pro Trp Val Lys Ser Pro Thr Gly
65                  70                  75                  80

Ser Trp Gln Lys Glu Ala Glu Ile His Ser Lys Pro Val Phe Ile Thr
                85                  90                  95

Val Lys Met Asp Val Leu Asn Ala Phe Lys Tyr Pro
            100                 105


<210>  5
<211>  1049
<212>  DNA
<213>  Homo sapiens


<220>
<221>  misc_feature
<222>  (960)..(960)
<223>  n is a, c, g, or t

<220>
<221>  misc_feature
<222>  (963)..(963)
<223>  n is a, c, g, or t

<220>
<221>  misc_feature
<222>  (977)..(978)
<223>  n is a, c, g, or t

<220>
<221>  misc_feature
<222>  (981)..(981)
<223>  n is a, c, g, or t

<220>
<221>  misc_feature
<222>  (994)..(996)
<223>  n is a, c, g, or t

<220>
<221>  misc_feature
<222>  (998)..(998)

<223>  n is a, c, g, or t

<220>
<221>  misc_feature
<222>  (1012)..(1013)
<223>  n is a, c, g, or t

<220>
<221>  misc_feature
<222>  (1019)..(1019)
<223>  n is a, c, g, or t

<220>
<221>  misc_feature
<222>  (1023)..(1023)
<223>  n is a, c, g, or t

<220>
<221>  misc_feature
<222>  (1029)..(1029)
<223>  n is a, c, g, or t

<220>
<221>  misc_feature
<222>  (1036)..(1036)
<223>  n is a, c, g, or t

<220>
<221>  misc_feature
<222>  (1047)..(1047)
<223>  n is a, c, g, or t

<400>  5
   ttcccctcta gaaataattt tgtttaactt taagaaggag atatacatat gcaccatcat      60
   catcatcatt cttctggtct ggtgccacgc ggttctggta tgaaagaaac cgctgctgct     120
   aaattcgaac gccagcacat ggacagccca gatctgggta ccgatgacga cgacaagatg     180
   gcctttgatg tgtcctggtt tgcggtgcac tcttttggcc tggacaaggc tcctgtgctc     240
   ctgtcttccc tggatcggaa gggcatcgtg accacctccc ggagggactg gaagagcgac     300
   ctcagcctgg agcgcgtgag tgtgctggaa ttcttgctgc aagtgcatgg ctccgaggac     360
   caggactttg gcaactacta ctgttccgtg actccatggg tgaagtcacc aacaggttcc     420
   tggcagaagg aggcagagat ccactccaag cccgttttta taactgtgaa gatggatgtg     480
   ctgaacgcct tcaagtatcc ctgaaccggg cttctcctca accatggcga tatcggatcc     540
   gaattcgagc tccgtcgaca agcttgcggc cgcactcgag caccaccacc accaccactg     600
   agatccggct gctaacaaag cccgaaagga agctgagttg gctgctgcca ccgctgagca     660
   ataactagca taaccccttg gggcctctaa acgggtcttg aggggttttt tgctgaaagg     720
   aggaactata tccggattgg cgaatgggac gcgccctgta gcggcgcatt aagcgcggcg     780
   ggtgtggtgg ttacgcgcag cgtgaccgct acacttgcca gcgccctagc gcccgctctt     840
   tcgctttctt ccttcctttc tcgccacgtt cgccggcttt ccccgtcaag ctctaaatcg     900
   ggctcccttt agggttccga atttagtgct tacggccctc gaccccaaaa acttgattan     960
   ggngatggtt cacgtanngg ncatcccctg atannngntt ttcgcctttg anntggagnc    1020
   ccnttcttna tagggnacct tgttccnaa                                      1049

<210>  6
<211>  151
<212>  PRT
<213>  Homo sapiens

<400>  6

Met His His His His His His Ser Ser Gly Leu Val Pro Arg Gly Ser
1               5                   10                  15

Gly Met Lys Glu Thr Ala Ala Ala Lys Phe Glu Arg Gln His Met Asp
            20                  25                  30

Ser Pro Asp Leu Gly Thr Asp Asp Asp Asp Lys Met Ala Phe Asp Val
        35                  40                  45

Ser Trp Phe Ala Val His Ser Phe Gly Leu Asp Lys Ala Pro Val Leu
    50                  55                  60

Leu Ser Ser Leu Asp Arg Lys Gly Ile Val Thr Thr Ser Arg Arg Asp
65                  70                  75                  80

Trp Lys Ser Asp Leu Ser Leu Glu Arg Val Ser Val Leu Glu Phe Leu
                85                  90                  95

Leu Gln Val His Gly Ser Glu Asp Gln Asp Phe Gly Asn Tyr Tyr Cys
            100                 105                 110

Ser Val Thr Pro Trp Val Lys Ser Pro Thr Gly Ser Trp Gln Lys Glu
        115                 120                 125

Ala Glu Ile His Ser Lys Pro Val Phe Ile Thr Val Lys Met Asp Val
    130                 135                 140

Leu Asn Ala Phe Lys Tyr Pro
145                 150


<210>  7
<211>  90
<212>  PRT
<213>  Homo sapiens

<400>  7

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn Tyr Tyr Cys Ser Val Thr Pro Trp Val Lys Ser Pro Thr Gly
65                  70                  75                  80

Ser Trp Gln Lys Glu Ala Glu Ile His Ser
                85                  90

<210>  8
<211>  77
<212>  PRT
<213>  Homo sapiens

<400>  8

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn Tyr Tyr Cys Ser Val Thr Pro Trp Val Lys Ser
65                  70                  75

<210>  9
<211>  66
<212>  PRT
<213>  Homo sapiens

<400>  9

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu Phe Leu Leu Gln Val His Gly Ser Glu Asp Gln Asp Phe
    50                  55                  60

Gly Asn
65

<210>  10
<211>  51
<212>  PRT
<213>  Homo sapiens

<400>  10

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp Trp Lys Ser Asp Leu Ser Leu Glu Arg Val Ser
        35                  40                  45

Val Leu Glu
    50

<210>  11
<211>  37
<212>  PRT
<213>  Homo sapiens

<400>  11

Met Ala Phe Asp Val Ser Trp Phe Ala Val His Ser Phe Gly Leu Asp
1               5                   10                  15

Lys Ala Pro Val Leu Leu Ser Ser Leu Asp Arg Lys Gly Ile Val Thr
            20                  25                  30

Thr Ser Arg Arg Asp
        35

<210>  12
<211>  20
<212>  PRT
<213>  Homo sapiens

<400>  12

Met Ala Phe Asp Val Ser Trp Phe Ala Val His Ser Phe Gly Leu Asp
1               5                   10                  15

Lys Ala Pro Val
            20

<210>  13
<211>  270
<212>  DNA
<213>  Homo sapiens

<400>  13
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaacta ctactgttcc gtgactccat gggtgaagtc accaacaggt     240

tcctggcaga aggaggcaga gatccactcc                                      270

<210>  14
<211>  231
<212>  DNA
<213>  Homo sapiens

<400>  14
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaacta ctactgttcc gtgactccat gggtgaagtc a              231


<210>  15
<211>  198
<212>  DNA
<213>  Homo sapiens

<400>  15
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaattcttgc tgcaagtgca tggctccgag     180

gaccaggact ttggcaac                                                   198


<210>  16
<211>  153
<212>  DNA
<213>  Homo sapiens

<400>  16
atggcctttg atgtgtcctg gtttgcggtg cactcttttg gcctggacaa ggctcctgtg      60

ctcctgtctt ccctggatcg gaagggcatc gtgaccacct cccggaggga ctggaagagc     120

gacctcagcc tggagcgcgt gagtgtgctg gaa                                  153


<210>  17
<211>  111
<212>  DNA
<213>  Homo sapiens

<400>  17
atggcctttg atgtgtcctg gtttgcggtg cactcttttg gcctggacaa ggctcctgtg      60

ctcctgtctt ccctggatcg gaagggcatc gtgaccacct cccggaggga c              111


<210>  18
<211>  60
<212>  DNA
<213>  Homo sapiens

<400>  18
atggcctttg atgtgtcctg gtttgcggtg cactcttttg gcctggacaa ggctcctgtg      60